Europäisches Patentamt

European Patent Office

Office européen des brevets

(19)

(11) **EP 1 012 359 B1**

(12) **EUROPÄISCHE PATENTSCHRIFT**

(45) Veröffentlichungstag und Bekanntmachung des Hinweises auf die Patenterteilung:
**02.05.2003 Patentblatt 2003/18**

(51) Int Cl.7: **D01F 1/00**

(21) Anmeldenummer: **98945181.0**

(22) Anmeldetag: **07.08.1998**

(86) Internationale Anmeldenummer:
**PCT/EP98/05030**

(87) Internationale Veröffentlichungsnummer:
**WO 99/007926 (18.02.1999 Gazette 1999/07)**

(54) **VERFAHREN ZUM HERSTELLEN EINER CELLULOSEFASER AUS HYDRATCELLULOSE**

METHOD FOR PRODUCING A CELLULOSE FIBRE FROM HYDROCELLULOSE

PROCEDE PERMETTANT DE FABRIQUER UNE FIBRE DE CELLULOSE A PARTIR D'HYDROCELLULOSE

(84) Benannte Vertragsstaaten:
**AT BE CH CY DE DK ES FI FR GB GR IE IT LI LU MC NL PT SE**

(30) Priorität: **07.08.1997 DE 19734239**
**06.03.1998 DE 19809765**

(43) Veröffentlichungstag der Anmeldung:
**28.06.2000 Patentblatt 2000/26**

(73) Patentinhaber:
• **Neumayr, Achim**
**89444 Villenbach (DE)**
• **Hasl, Herbert**
**82487 Oberammergau (DE)**

(72) Erfinder:
• **Neumayr, Achim**
**89444 Villenbach (DE)**
• **Hasl, Herbert**
**82487 Oberammergau (DE)**

(74) Vertreter: **Grünecker, Kinkeldey,**
**Stockmair & Schwanhäusser Anwaltssozietät**
**Maximilianstrasse 58**
**80538 München (DE)**

(56) Entgegenhaltungen:
EP-A- 0 574 762     DE-U- 29 801 027
GB-A- 133 047       GB-A- 369 912
GB-A- 757 233       GB-A- 761 511
US-A- 2 046 670

• SEREBRYAKOVA Z G ET AL: "SURFACTANTS AND MODIFIERS IN PRODUCTION OF VISCOSE FIBRES (REVIEW)" FIBRE CHEMISTRY, Bd. 28, Nr. 2, März 1996, Seiten 91-94, XP000644131
• DATABASE WPI Section Ch, Week 9101 Derwent Publications Ltd., London, GB; Class A18, AN 91-002394 XP002086973 & JP 02 277846 A (ASAHI CHEM IND CO LTD) , 14. November 1990

**Beschreibung**

Diese Erfindung betrifft ein Verfahren zum Herstellen einer Cellulosefaser aus Hydratcellulose, eine nach diesem Verfahren erhältliche Cellulosefaser, ein Gewebe, das diese Cellulosefaser enthält, und Verwendungen dieses Gewebes.

Saugfähige Fasermaterialien, die auch zu Reinigungszwecken verwendet werden können, sind bereits bekannt. Beispiele dafür sind vernetzte Carboxymethylcellulose, die nach dem in der CH-A-491140 beschriebenen Verfahren hergestellt werden kann, oder Viskosefasern, die hydrophile polymere Substanzen, wie Polyacrylsäure (BE-A-2324589), Poly-N-Vinylpyrrolidon oder Carboxymethylcellulose (DE-A-25 50 345), Alginsäure (DE-A-27 50 622) oder andere Copolymere (DE-A-27 50 900), enthalten. Diese Fasern haben neben einer hohen Saugfähigkeit auch ein hohes Wasserrückhaltevermögen. Die Herstellung dieser Fasern erfordert jedoch einen erheblichen verfahrenstechnischen Aufwand, und die Fasern enthalten teilweise biologisch nicht oder nur schwer abbaubare Substanzen, so daß eine natürliche Entsorgung (z.B. Kompostierung) der Fasern nach deren Verwendung nicht möglich ist.

Aufgabe der vorliegenden Erfindung ist es, ein Verfahren zum Herstellen einer biologisch leicht abbaubaren Cellulosefaser aus Hydratcellulose mit einer extrem großen Oberfläche bereitzustellen. Eine andere Aufgabe der Erfindung ist es, ein Gewebe unter Verwendung dieser Faser bereitzustellen, das sich durch eine hohe Saugfähigkeit, ein hohes Flüssigkeitsrückhaltevermögen, ein hohes Fettlösungsvermögen sowie durch Partikel adsorbierende Eigenschaften auszeichnet, das zur Herstellung leicht zu reinigender Produkte geeignet ist, das zum Reinigen und Dekontaminieren sowie zur Verringerung der Oberflächenspannung von Wasser verwendet werden kann und das umweltfreundlich entsorgt werden kann.

Die zuvor genannten Aufgaben werden erfindungsgemäß durch die Bereitstellung eines Verfahrens zum Herstellen einer Cellutosefaser aus Hydratcellulose, das die folgenden Schritte umfaßt:

a) das Behandeln von Zellstoff aus höchstens 1-jährigen Schößlingen von Lauboder Nadelhölzern mit einer Alkalimetallhydroxidlösung, um eine Alkalicellulose zu erhalten,

b) das Abpressen der überschüssigen Alkalimetallhydroxidlösung aus der erhaltenen Alkalicellulose,

c) das Zerfasern der Alkalicellulose,

d) das Vorreifen der zerfaserten Alkalicellulose bis zu einer Reife von 5° bis 30° Hottenroth,

e) das Behandeln der vorgereiften Alkalicellulose nach dem Naßsulfidverfahren, um die Cellulose zu xanthogenieren,

f) das Spülen und Verdünnen der xanthogenierten Cellulose mit Wasser, um eine Spinnlösung herzustellen,

g) das Nachreifen der gespülten und verdünnten Cellulose bis zu einer Reife von 5° bis 30° Hottenroth,

h) das Filtrieren und anschließende Entlüften der Spinnlösung,

i) das Einpressen der Spinnlösung in ein Fällbad unter Verwendung von Spinndüsen,

j) das Abziehen der koagulierenden Fasern unter gleichzeitigem Verdrillen der Fasern, um eine verdrillte Faser herzustellen,

k) das Entwässern der verdrillten Faser,

l) das Entschwefeln der verdrillten Faser,

m) das Waschen der verdrillten Faser mit Wasser,

n) das Vorentwässern der verdrillten Faser und

o) das Trocknen der verdrillten Faser,

und durch ein Gewebe, umfassend ein Grundgewebe und einen darin eingewobenen Flor, der diese Faser enthält, gelöst.

Die Figuren 1 bis 6 zeigen die Mikrostruktur von erfindungsgemäßen Fasern.

Die Figuren 7 bis 15 zeigen die Makrostruktur von erfindungsgemäßen Fasern.

Die Figur 16 zeigt ein Beispiel für das erfindungsgemäße Gewebe.

Die Figuren 17 bis 20 zeigen elektronenmikroskopische Aufnahmen von Bakterien, die an die Lamellen der erfindungsgemäßen Faser adsorbiert sind.

Die Figur 21 zeigt eine Vorrichtung zur Gewinnung von Wasser mit verringerter Oberflächenspannung.

Die Figur 22 zeigt ein Tensiometer zur Messung der Oberflächenspannung.

Die Figuren 23 bis 28 zeigen die oberflächenentspannende Wirkung von erfindungsgemäßen Geweben in Abhängigkeit der Spülungen der Gewebe.

Die Figur 29 zeigt den zeitlichen Verlauf der Oberflächenspannung mit im Wasser verbleibenden Geweben.

Die Figur 30 zeigt die oberflächenentspannende Wirkung des erfindungsgemäßen Gewebes vor bzw. nach einer Trocknungsphase.

Die Figur 31 zeigt einen Versuchsaufbau zur Messung des Wasseraufnahmevermögens des erfindungsgemäßen Gewebes.

Die Figur 32 zeigt ein Modell zur Berechnung der spezifischen Oberfläche des erfindungsgemäßen Gewebes.

Das erfindungsgemäße Verfahren ermöglicht die Herstellung einer biologisch leicht abbaubaren Cellulosefaser aus Hydratcellulose $(C_6H_{10}O_5)_n$, deren Mikrostruktur faserparallele Lamellen aufweist. Der Lamellenabstand liegt bevorzugt im Bereich von 1 nm bis 5 µm und besonders bevorzugt im Bereich von 200 nm bis 1 µm. Ein weiterer besonders bevorzugter Bereich ist 1 bis 20 nm. Infolge dieser Mikrostruktur besitzt die Faser eine extrem große Oberfläche. In einer bevorzugten Ausführungsform ist die erfindungsgemäße Faser doppelt lichtbrechend. Die Mikrostruktur sowie die Makrostruktur der erfindungsgemäßen Fasern wurden mit den im folgenden beschriebenen Verfahren bestimmt.

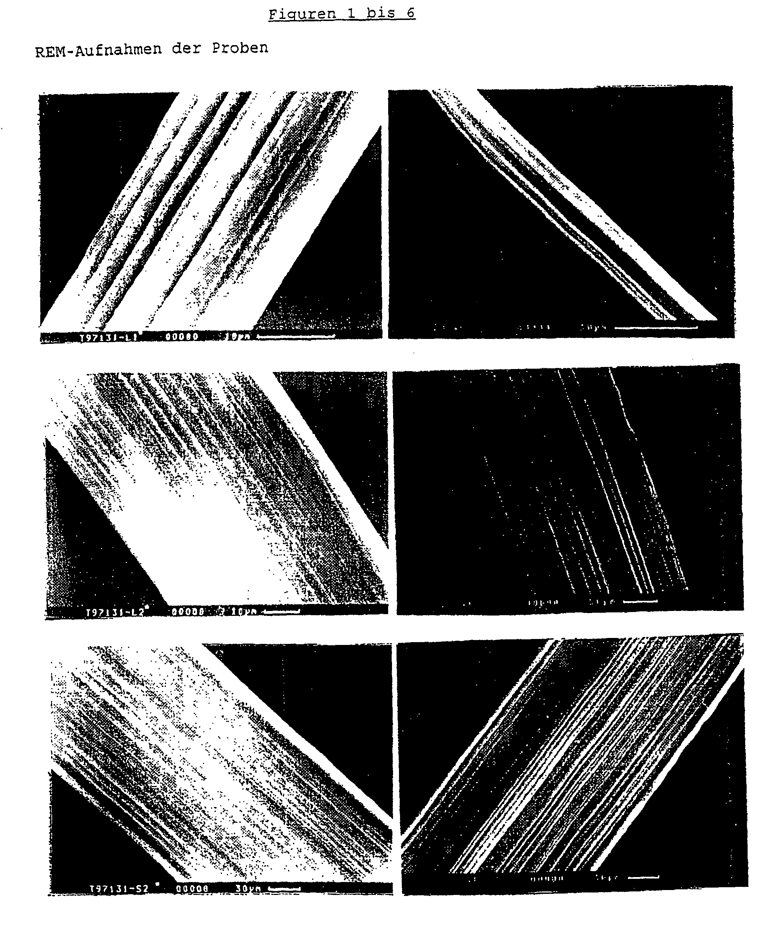

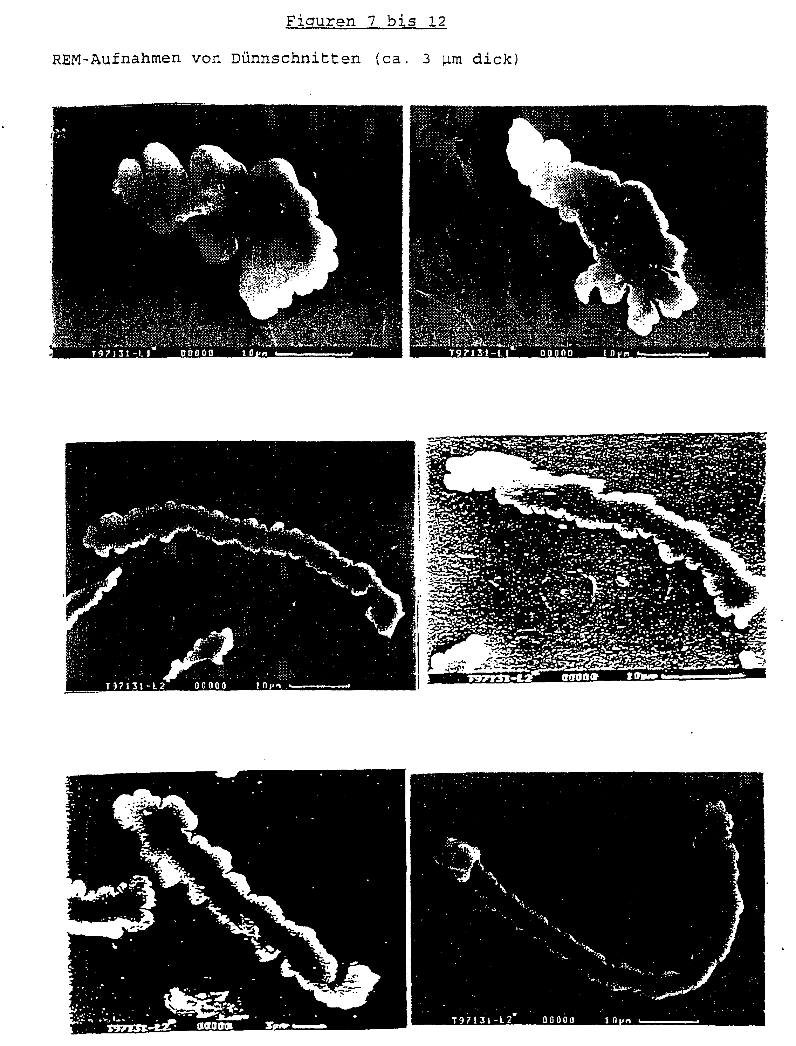

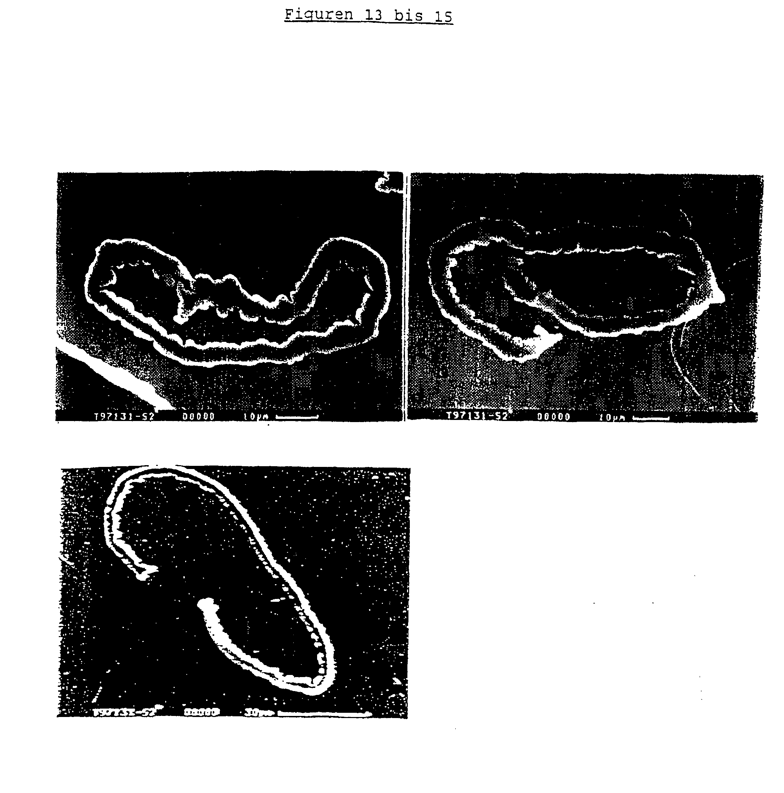

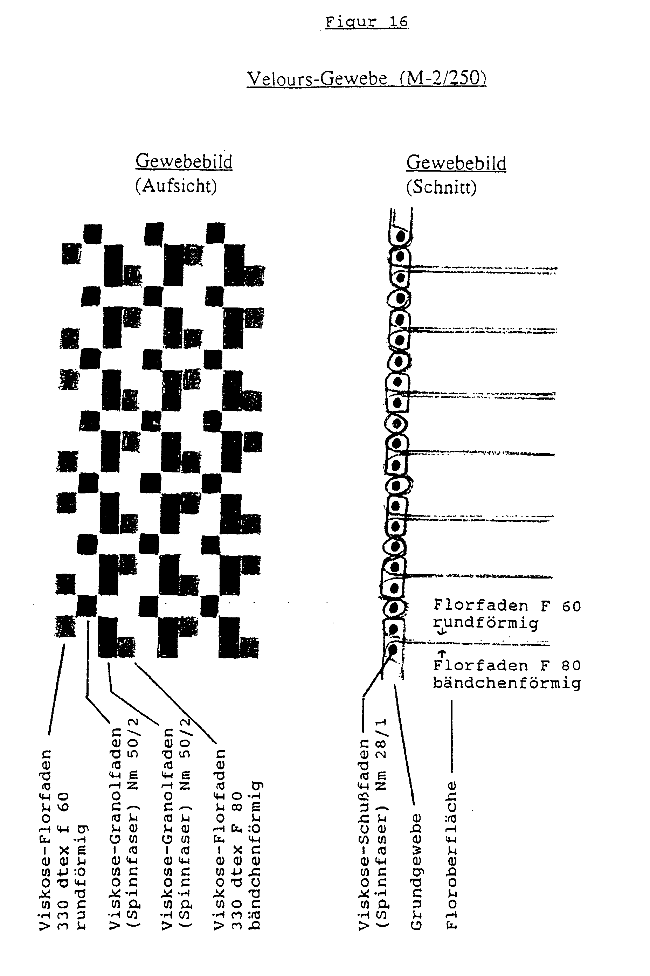

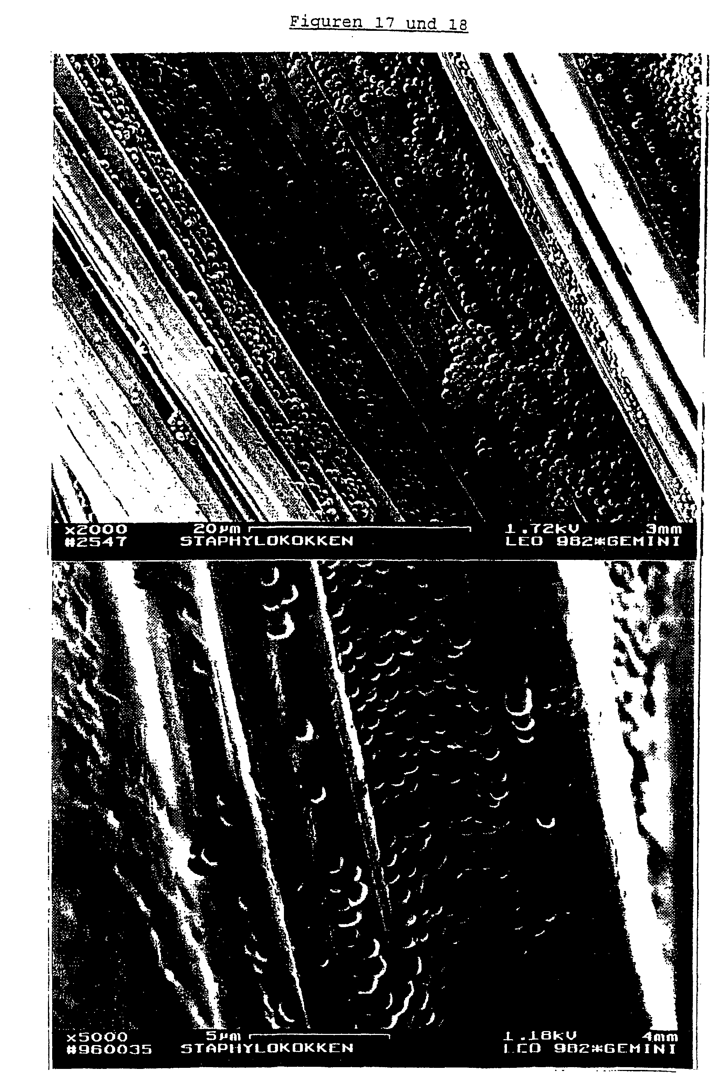

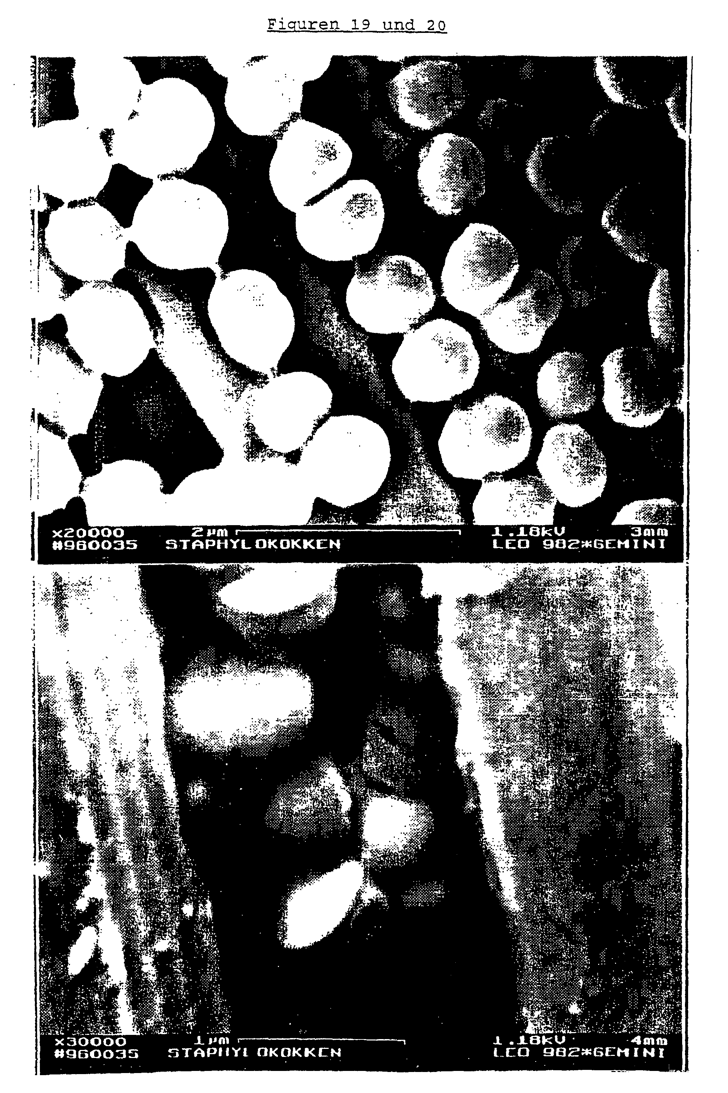

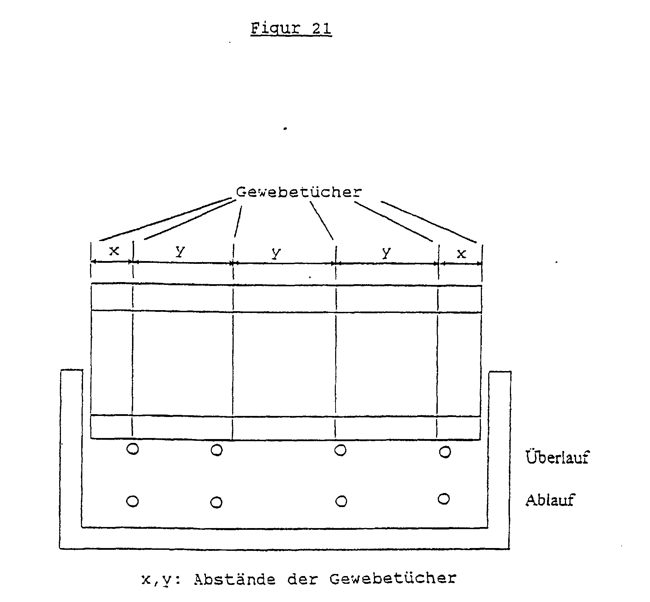

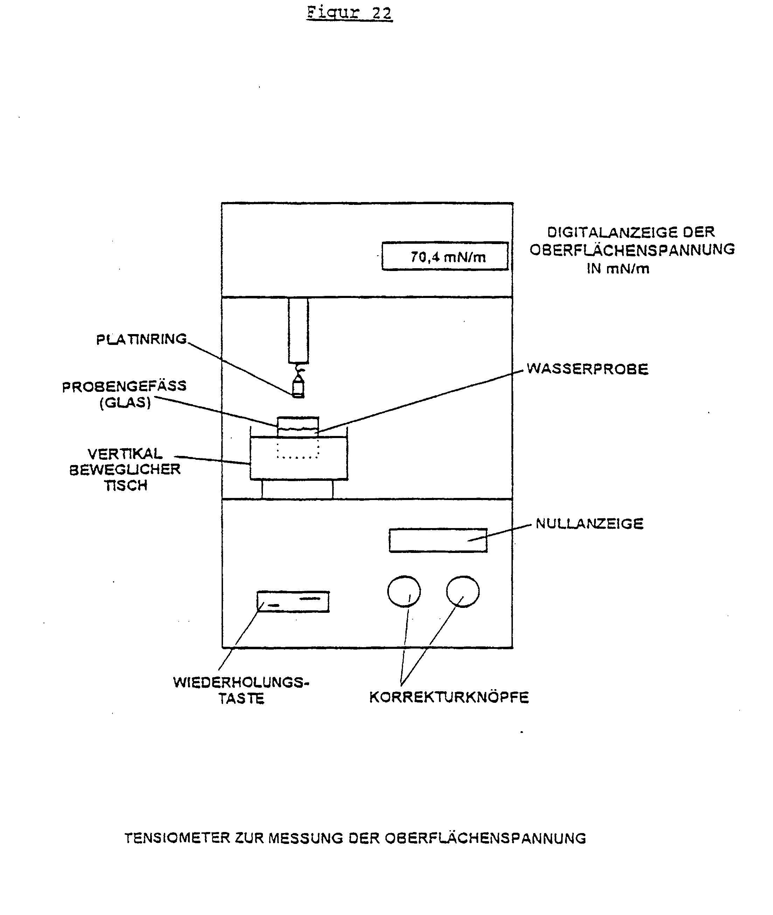

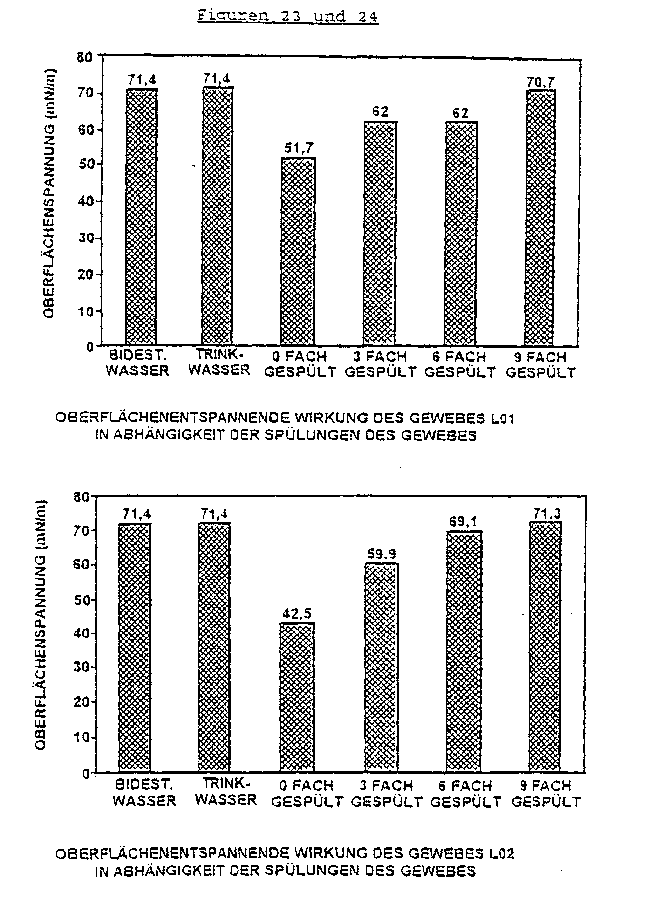

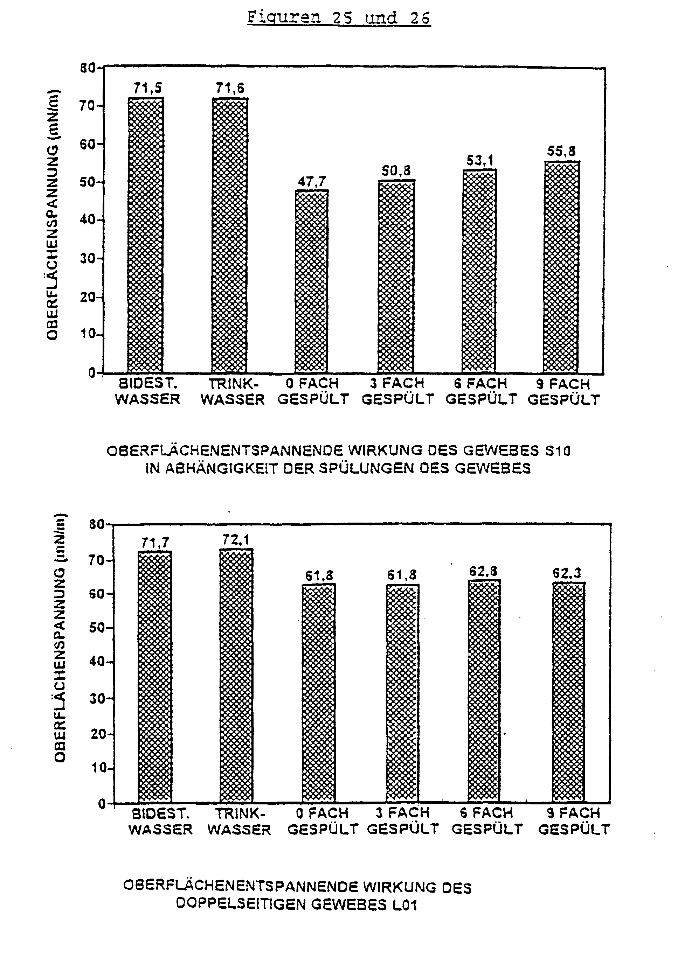

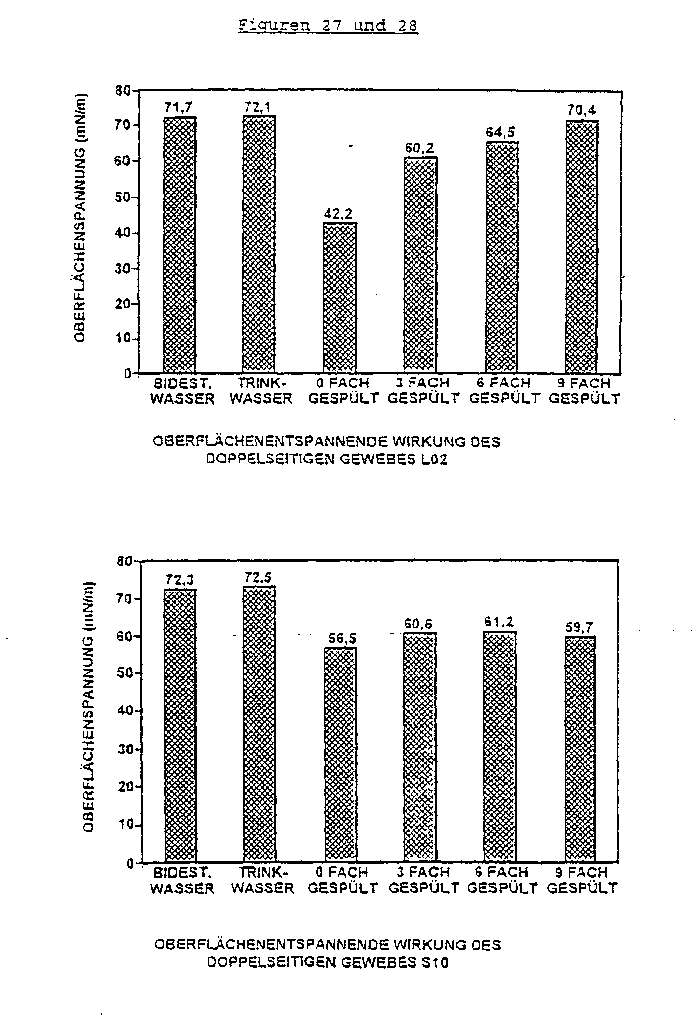

Die Mikrostruktur von verschiedenen erfindungsgemäßen Fasern, die in den durchgeführten Untersuchungen mit L1, L2 und S2 bezeichnet wurden, ist in den Figuren 1 bis 6 gezeigt. Bei diesen Untersuchungen wurden die Fasern als gesamte Fasern hinsichtlich der Oberflächenstruktur im Rasterelektronenmikroskop untersucht. Die Makrostruktur der Fasern, die in den Figuren 7 bis 15 gezeigt ist, wurde im Mikrotomschnitt untersucht. Zur Erstellung der Mikrotomschnitte wurden die Fasern in PMMA eingebettet, geschnitten und danach wieder aus dem Einbettmittel herausgelöst. Die Temperaturbelastung der Fasern wurde dabei so gering wie möglich gehalten.

Die Figuren 1 bis 6 zeigen die Mikrostrukturen, die in der Aufsicht erhalten wurden. Alle erfindungsgemäßen Fasern besitzen eine faserparallele Lamellenstruktur. Aus diesen Figuren können jedoch keine Hinweise auf die Querschnittsstruktur (Makrostruktur) gewonnen werden. Erst die Mikrotomschnitte zeigen die Querschnittsstrukturen, die in den Figuren 7 bis 15 dargestellt sind. Die Faser L1 wurde unter Verwendung einer ovalen Spinndüse hergestellt, während die Fasern L2 und S2 unter Verwendung einer langgestreckt-schlitzförmigen Spinndüse hergestellt wurden. Die qualitativen Ergebnisse der Untersuchungen sind in der folgenden Tabelle 1 zusammengestellt.

TABELLE 1

| Fasertyp | L1 | L2 | S2 |
|---|---|---|---|
| Querschnittsform (Makrostruktur) | globular, stark zerklüftet | lamellar, zerklüftet | lamellar, zerklüftet |
| Spez. Oberfläche (qualitativ) | sehr groß | mittelgroß | groß |
| Lage der Oberflächenstruktur (Einschnitte) | allseitig | allseitig | überwiegend einseitig, und zwar innen nach dem Einrollen |
| Faserbreite max [um] | ca. 35 | ca. 80 | ca. 200 |
| Einrolleffekt nach dem Schneiden (Hinweis auf Eigenspannungen) | nicht feststellbar | gering | hoch |

Zum Herstellen der erfindungsgemäßen Faser wird ein Zellstoff aus höchstens einjährigen Schößlingen von Laub- oder Nadelhölzern verwendet. Aus solch einem Ausgangsmaterial läßt sich Lignin besonders leicht entfernen. Bevorzugt wird Zellstoff aus höchstens 1-jährigen Schößlingen von Robiniengehölz, Teakholz, Bongassiholz oder Bambus verwendet, es kann jedoch auch Zellstoff aus höchstens 1-jährigen Schößlingen vergleichbarer europäischer Hölzer verwendet werden. Der Ligningehalt der verwendeten höchstens 1-jährigen Schößlinge sollte so gering wie möglich sein und beträgt bevorzugt nicht mehr als 7%. In einer besonders bevorzugten Ausführungsform der Erfindung beträgt der Ligningehalt des Ausgangsmaterials nicht mehr als 5%, besonders bevorzugt beträgt er nicht mehr als 2%.

Dieser Zellstoff wird mit einer Alkalimetallhydroxidlösung behandelt, vorzugsweise bei einer Temperatur im Bereich von 15 bis 25°C, um eine Alkalicellulose herzustellen. Es ist bevorzugt, daß eine Natronlauge mit einem Gehalt an Natriumhydroxid im Bereich von 150 bis 350 g/l als Alkalimetallhydroxidlösung verwendet wird. Ein Gehalt an Natriumhydroxid von etwa 300 g/l ist besonders bevorzugt.

Aus der erhaltenen Alkalicellulose wird dann die überschüssige Alkalimetallhydroxidlösung abgepreßt, z.B. unter Verwendung einer Tauchpresse.

Danach wird die Alkalicellulose zerfasert, wobei das Zerfasern einen Grobzerkleinerungsschritt (z.B. in einem Vorzerreißer) und einen Feinzerkleinerungsschritt (z.B. in einer Scheibenmühle) umfassen kann.

Die zerfaserte Alkalicellulose wird dann z.B. einer Reifetrommel zugeführt und bis zu einer Reife von 5° bis 30° Hottenroth, bevorzugt bis zu einer Reife von 8° bis 12° Hottenroth und besonders bevorzugt bis zu einer Reife von etwa 10° Hottenroth vorgereift. Die Temperatur beim Vorreifen beträgt bevorzugt 60 bis 80°C, besonders bevorzugt 65 bis 75°C und ganz besonders bevorzugt etwa 72°C. Anschließend kann der Vorreifeprozeß durch Senkung der Temperatur auf 40 bis 50°C, bevorzugt auf etwa 45°C, abgebremst werden.

Die vorgereifte Alkalicellulose wird dann entsprechend dem gewöhnlichen Naßsulfidverfahren weiterverarbeitet, um die Cellulose zu xanthogenieren. Das Naßsulfidverfahren wird bevorzugt in einer Lösung mit Schwefelkohlenstoff, Natriumhydroxid und Berol durchgeführt. Dabei beträgt der Gehalt an Schwefelkohlenstoff bevorzugt 150 bis 250 g/l, besonders bevorzugt 180 bis 210 g/l, der Gehalt an Natriumhydroxid beträgt bevorzugt 250 bis 350 g/l, besonders bevorzugt 280 bis 320 g/l, und der Gehalt an Berol beträgt bevorzugt 100 bis 200 g/l, besonders bevorzugt etwa 150 g/l. Das für diesen Verfahrensschritt verwendete Berol ist bevorzugt ein handelsüblich erhältliches Produkt der Firma Berol-Kemie, 44401 Stennungsund/Schweden.

Nach dem Xanthogenieren erfolgt das Spülen und Verdünnen der xanthogenierten Cellulose mit Wasser, um eine Spinnlösung herzustellen. Danach wird die Cellulose bis zu einer Reife von 5° bis 30° Hottenroth, bevorzugt bis zu einer Reife von 8° bis 12° Hottenroth, nachgereift. Der beim Vorreifen erreichte Reifegrad wird durch die Xanthogenierung der Cellulose und das Spülen und Verdünnen der xanthogenierten Cellulose zunächst wieder vermindert, so daß eine Nachreifung erst wieder den gewünschten Reifegrad erbringt. In der Praxis kann es sich als schwierig erweisen, den Reifegrad exakt zu steuern. In diesem Fall können zwei oder mehrere Chargen von Spinnlösungen miteinander vermischt werden, um den gewünschten Reifegrad einzustellen.

Das nachträgliche Filtrieren der Spinnlösung erfolgt z.B. unter Verwendung von Filterpressen. Dann wird die Spinnlösung entlüftet.

Die entlüftete Spinnlösung wird unter Verwendung von Spinndüsen in ein Fällbad bei einer Temperatur von bevorzugt 35 bis 45°C, besonders bevorzugt bei einer Temperatur von etwa 40°C, eingebracht. Ein geeignetes Fällbad enthält 70 bis 160 g/l, bevorzugt 90 bis 140 g/l und besonders bevorzugt etwa 120 g/l Schwefelsäure, 0,3 bis 4 g/l, bevorzugt 0,5 bis 2 g/l und besonders bevorzugt etwa 1 g/l Zinksulfat, sowie 0,05 bis 1 g/l, bevorzugt 0,1 bis 0,7 g/l und besonders bevorzugt etwa 0,4 g/l Berol. Das für diesen Verfahrensschritt verwendete Berol ist bevorzugt ein handelsüblich erhältliches Produkt der Firma Berol-Kemie, 44401 Stennungsund/Schweden. Die verwendeten Spinndüsen, die oval bis langgestreckt schlitzförmig sein können, werden durch Beheizen auf einer Temperatur im Bereich von bevorzugt 55 bis 75°C, besonders auf einer Temperatur im Bereich von 65 bis 70°C und ganz besonders bevorzugt auf einer Temperatur von etwa 67°C gehalten.

Die koagulierenden Fasern werden unter gleichzeitigem Verdrillen der Fasern abgezogen, um eine verdrillte Faser herzustellen, die nachfolgend entwässert wird. Zum Entwässern kann z.B. eine Schwefelsäurelösung verwendet werden, die bevorzugt bis zu 15 g/l und besonders bevorzugt bis zu 10 g/l Schwefelsäure enthält.

Die Entschwefelung der verdrillten Faser wird üblicherweise in einer Natriumsulfatlösung durchgeführt, die bevorzugt 2 bis 5 g/l und besonders bevorzugt etwa 3 g/l Natriumsulfat enthält. Andere Entschwefelungsverfahren sind ebenfalls möglich. Danach wird die Faser mit Wasser gewaschen.

Nach dem Waschen der Faser kann die Faser weiterhin präpariert werden, um z.B. die optischen Eigenschaften der Faser zu modifizieren. Das Präparieren kann z.B. mit Titandioxid durchgeführt werden, um der Faser ein mattes Aussehen zu verleihen.

Dann wird die Faser vorentwässert und getrocknet. Dabei sollte die Faser so wenig wie möglich mechanisch belastet werden, um die Lamellenstruktur der Faser nicht zu zerstören. Das Vorentwässern kann z.B. unter Verwendung von Druckluft, und das Trocknen z.B. unter Verwendung von Kanaltrocknern erfolgen, obwohl für diesen Zweck auch andere geeignete Verfahren und Vorrichtungen verwendet werden können, die dem Fachmann bekannt sind.

Mit dem erfindungsgemäßen Herstellungsverfahren wird eine Faser erhalten, die im wesentlichen kein Lignin mehr enthält und die weitgehend frei von Schwefelsäure und Schwefelkohlenstoff ist. Diese Faser besitzt infolge ihrer lamellenförmigen Mikrostruktur eine extrem große Oberfläche. Eine solch große Oberfläche kann bei üblichen Fasern, die aus Sulfit-Zellstoff hergestellt werden, nicht erreicht werden, da das Sulfit-Verfahren zu einer Zerstörung der Lamellenstruktur führt.

Die auf diese Weise erhaltene Faser hat bevorzugt einen Titer von 330 dtex oder mehr.

Die wie zuvor beschrieben erhaltene Faser kann zum Herstellen eines Gewebes verwendet werden, das sich durch eine hohe Saugfähigkeit, ein hohes Flüssigkeitsrückhaltevermögen, ein hohes Fettlösungsvermögen und durch Partikel adsorbierende Eigenschaften auszeichnet.

Das erfindungsgemäße Gewebe umfaßt ein Grundgewebe und einen darin eingewobenen Flor, der die Faser enthält, die entsprechend dem zuvor beschriebenen Verfahren hergestellt wurde.

Das Grundgewebe besitzt bevorzugt eine gitterförmige Struktur. Das Grundgewebe und der Flor können, müssen aber nicht aus der gleichen Faserart bestehen. Haltbarkeitskriterien können z.B. stärkere Grundgewebefasern erfordern. Es ist bevorzugt, daß das Grundgewebe Viskose-Spinnfasern enthält; besonders bevorzugt besteht das Grundgewebe ausschließlich aus solchen Fasern.

In einer bevorzugten Ausführungsform der Erfindung bildet der Flor einen Faserrasen von etwa 0,5 cm Länge zum Grundgewebe. Es ist bevorzugt, daß der Flor ovale Fasern, bändchenförmige Fasern oder eine Mischung dieser Fasern umfaßt. Ein Gewebe, dessen Flor viele ovale Fasern, aber nur wenig bändchenförmige Fasern umfaßt, besitzt insbesondere fettadsorbierende Eigenschaften. Ein Gewebe, dessen Flor wenig ovale Fasern, aber viele bändchenförmige Fasern umfaßt, eignet sich insbesondere zur Verringerung der Oberflächenspannung von Wasser.

In einer weiteren bevorzugten Ausführungsform der Erfindung besteht der Flor aus 50% ovalen und 50% bändchenförmigen Fasern. In einer besonders bevorzugten Ausführungsform besteht der Flor aus je 50% Fasern der Dimension 330 dtex F 60 oval und 330 dtex F 80 bändchenförmig. Ein solches Gewebe ist in Figur 16 gezeigt.

Das erfindungsgemäße Gewebe zeichnet sich insbesondere durch die folgenden Eigenschaften aus:

- es kann Bakterien und Schmutzpartikel reversibel binden und danach chemiefrei gereinigt werden,

- es besitzt eine sehr große spezifische Oberfläche,

- es besitzt ein hohes spezifisches Flüssigkeitsbindevermögen und

- es kann umweltfreundlich entsorgt werden.

Aufgrund der zuvor genannten Eigenschaften ergeben sich vielfältige Einsatzmöglichkeiten für das erfindungsgemäße Gewebe.

Da das erfindungsgemäße Gewebe pH-neutral ist, kann es z.B. zur Herstellung von hautverträglichen Hygieneartikeln, wie z.B. Monatsbinden, als Kosmetiktuch zur Körperpflege und Hautreinigung, insbesondere zur Reinigung der Schleimhaut (Stoma-Reinigung), zur begleitenden Hautreinigung bei Hautinfektionskrankheiten und bei Neurodermitis sowie als Hautreinigungstuch im Bereich der Altenpflege verwendet werden. Bei der Verwendung als Hautreinigungstuch können ohne Verwendung von Hautreinigungsmitteln wasserlösliche und fetthaltige Kosmetika allergenfrei und pH-neutral von der Hautoberfläche entfernt werden. Das Gewebe kann anschließend durch einfache mechanische Reinigung (Ausspülen und Ausdrücken) in kaltem Wasser bakterienfrei und kosmetikafrei gemacht werden. Besondere Relevanz erhält dieser Aspekt bei empfindlicher oder geschädigter Körperhaut, z.B. bei Neurodermitis oder bei Akne. Durch die Verwendung des erfindungsgemäßen Gewebes nur in Verbindung mit Wasser ist jede zusätzliche Reizung oder Schädigung der Haut ausgeschlossen. Keime können so z.B. besser von der Haut beseitigt werden als mit waschaktiven Substanzen oder mit Desinfektionsmitteln.

Das erfindungsgemäße Gewebe kann weiterhin zum Reinigen und Dekontaminieren, z.B als Eutertuch zum Reinigen und Dekontaminieren von Kuheutern vor deren Anschluß an die Melkmaschine, verwendet werden. in die beim Melken erhaltene Milch werden somit keine Keime eingebracht.

Andererseits kann das erfindungsgemäße Gewebe selbst zur Herstellung von leicht zu reinigenden Produkten, wie z.B. Textilien und Bekleidungsmaterialien, verwendet werden; solche Produkte können selbst bei starker Verschmutzung durch z.B. Lebensmittelrückstände ohne die Anwendung von Chemikalien gereinigt werden. Beispiele für solche Produkte umfassen Bettwäsche, Tischwäsche, Berufskleidung oder Babyartikel (z.B. Babywindeln, Babytücher und Babylätzchen), Polsterbezüge für Möbel oder Autositze sowie Bezugsstoffe für sogenannte Plüschoder Kuscheltiere. Von diesen Produkten können Rückstände von z.B. Ketchup, Säften, Rotwein, Lippenstift oder Blut leicht durch Abspülen mit kaltem Wasser rückstandslos entfernt werden. Produkte wie z.B. Babywindeln, Babytücher oder Babylätzchen können nach Gebrauch durch mechanische Reinigung ohne Zusatz von Waschmitteln bei Temperaturen von bis zu 40°C rückstandsfrei gereinigt werden. Es ist auch möglich, die Produkte in die Waschmaschine zu geben und kalt oder bei einer Temperatur von bis zu 40°C im Spülgang ohne Zugabe von Waschmitteln zu reinigen. Anschließend können die Produkte an freier Luft oder auch im Trockner bis 40°C getrocknet werden.

Ein weiterer Einsatzbereich für das erfindungsgemäße Gewebe ist die Verwendung als Fußbodenbelag für Spezialräume (wie z.B. Feuchträume) oder hygienisch empfindliche Räume.

Das erfindungsgemäße Gewebe kann weiterhin als Kondensationskatalysator zur Kondensation von Wasserdampf oder Luftfeuchtigkeit, z.B. als Abdeckung von Dusch- oder Badekabinen oder Feuchträumen, verwendet werden. Dabei wird die Feuchtigkeit aufgenommen und in einem langsamen Trocknungsvorgang wieder verzögert an die Raumluft abgegeben. Dadurch wird ein schnelles Kondensieren von Dampf oder Feuchtigkeit an den Raumwänden verhindert, selbst wenn der betreffende Raum schlecht belüftet wird (z.B. in Altbauten). Die Abdeckung aus dem erfindungsgemäßen Gewebe bleibt dabei pilz-, bakterien- und algenfrei.

Eine weitere Anwendungsmöglichkeit des erfindungsgemäßen Gewebes ist die Verwendung als Partikelfilter, z.B. zum Entfernen von Partikeln oder Mikroorganismen aus organischen und anorganischen Flüssigkeiten. Dabei kann das Gewebe z.B. in Form von parallelen Gewebeschichten oder in aufgerollter Form verwendet werden.

Die biophysikalischen Eigenschaften des erfindungsgemäßen Gewebes werden einerseits durch die lamellenförmige Mikrostruktur der Fasern und andererseits durch die Anordnung der Fasern an der Oberfläche des Gewebes hervorgerufen.

Im folgenden wird zuerst die Verwendung des erfindungsgemäßen Gewebes zum Reinigen und Dekontaminieren beschrieben. Diese Verwendung ermöglicht die Reinigung von organischen Oberflächen (wie z.B. der Haut) oder anorganischen Oberflächen (wie z.B. Gegenständen, Fußböden und Fenstern) sowie das Entfernen von Bakterien von diesen Oberflächen ohne Einsatz von Desinfektionsmitteln, so daß die zu reinigenden Oberflächen nicht chemisch oder thermisch belastet werden. Bei der Dekontamination werden, im Vergleich mit der Dekontamination mit Desinfektionsmitteln, gleiche oder bessere keimverhindernde Effekte erzielt, ohne daß Selektionsprozesse durch chemische Resistenzen auftreten.

Das erfindungsgemäße Gewebe wird mit Wasser benetzt, so daß es eine gewisse Restfeuchte (z.B. etwa 20%) aufweist. Bei diesem Restfeuchtegehalt kann dann die zu dekontaminierende Oberfläche mechanisch dekontaminiert werden, wobei die Eigenschaft des Gewebes, die Oberflächenspannung von Wasser herabzusetzen, zu einer verbesserten Lipidlöslichkeit führt. Mit einem Gewebe mit einer Fläche von etwa 600 cm$^2$ kann z.B. eine hochkontaminierte Fläche von 1 m$^2$ optimal gereinigt werden. Danach erfolgt die Dekontamination des Gewebes unter Wasserüberschuß und mechanischer Bewegung im Wasser. Durch diese Reinigung unter Wasserüberschuß tritt eine völlige Dekontamination des Gewebes unter Abgabe aller Partikel an das Aufnahmewasser auf. Das Gewebe selbst bleibt dabei biologisch und chemisch völlig inert. Eine Kontaminationssättigung des Reinigungswassers ist bei etwa 30 m$^2$ Reinigungsfläche für 10 l Wasser erreicht, jedoch abhängig vom Kontaminationsgrad der Fläche. Der bei etwa 20% Restfeuchte bestehende Adsorptionseffekt des Gewebes bleibt für jede Reinigung aufrechterhalten, solange keine unzulässigen chemischen, mechanischen oder thermischen Belastungen die Faserstruktur zerstören. Der optimale Verwendungsbereich des Gewebes liegt zwischen 5 und 30°C. Der Adsorptionseffekt wird bei Temperaturen von über 60°C zerstört. Der intensive Kontakt (Tränkung) mit Reinigungsmitteln führt zu einer Zerstörung der Funktion des Gewebes durch Schädigung der Oberflächenstruktur. Der Kontakt mit 0,1 normalen Säuren oder Laugen sowie mit alkoholischen Lösungen ist unproblematisch.

Die Figuren 17 bis 20 zeigen elektronenmikroskopische Aufnahmen (2000-, 5000-, 20000- und 30000-fache Vergrößerung) von Bakterien (hier Staphylokokken), die an die Lamellen der erfindungsgemäßen Faser adsorbiert sind.

Überraschenderweise hat sich gezeigt, daß mit dem erfindungsgemäßen Gewebe auch die Oberflächenspannung von Wasser, ohne Verwendung von Chemikalien, um z.B. 20% oder mehr verringert werden kann. Solch ein Wasser mit verringerter Oberflächenspannung kann bei Extraktions- und Syntheseprozessen sowie bei Fermentationsvorgängen eingesetzt werden, wie z.B. bei Brauvorgängen.

Zur Verringerung der Oberflächenspannung wird das Wasser mit dem erfindungsgemäßen Gewebe in Kontakt gebracht. Die Verringerung der Oberflächenspannung tritt nach kurzer Zeit ein, ist weitgehend unabhängig von der Temperatur des Wassers und hält etwa zwei Stunden lang an.

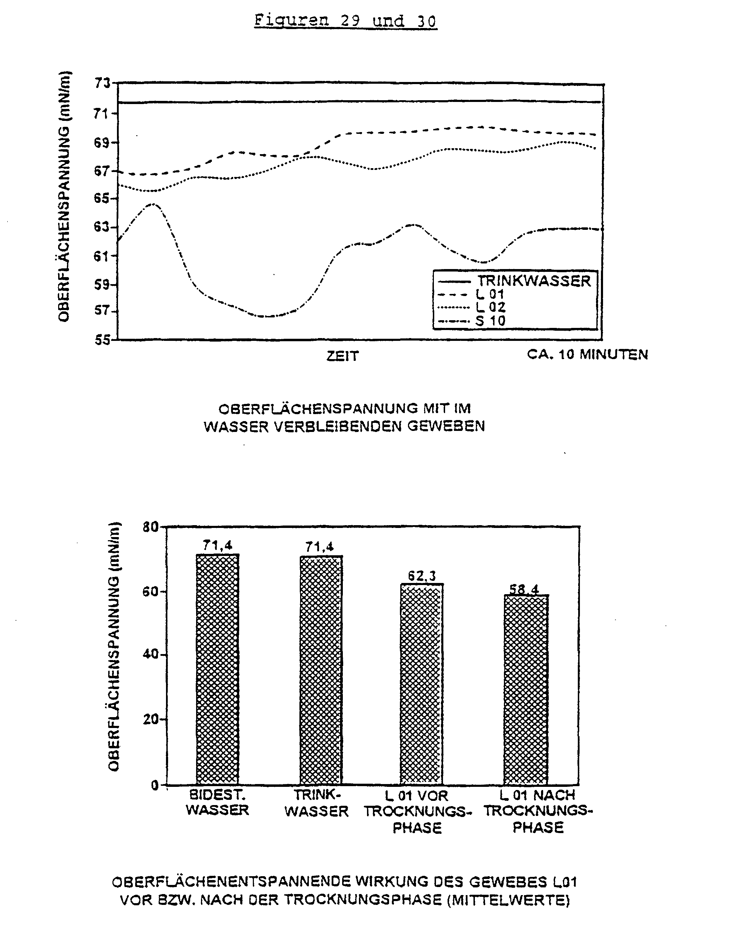

Figur 21 zeigt eine Vorrichtung zur Gewinnung von Wasser mit verringerter Oberflächenspannung. Die Vorrichtung umfaßt z.B. einen mit Wasser gefüllten Behälter, der mit einem Überlauf und einem Ablauf versehen ist. Es können mehrere erfindungsgemäße Gewebe gleichzeitig in den Behälter eingebracht werden.

Die folgenden Beispiele und die Figuren erläutern die Erfindung.

Beispiel 1: Gewebe zum Reinigen kontaminierter Oberflächen

Versuchsaufbau

1.1 Geprüfte Bakterienstämme

- E. coli
- Staphylokokkus aureus
- Streptokokkus pyogenes
- Enterokokken (S. fäkalis)
- Streptokokkus bovis

- Pseudomonas pyocyanea

1.2. Kultivierung

Inkubation in Nährbouillon oder Lackmusmilch (ein Gemisch aus Lackmus und Milch) bzw. Nähragar bei etwa 25° bis 30°, bis Keimzahl > $10^6$ Keime/ml. Die Anzahl der Keime wurde bestimmt (entspricht der primären Keimmenge = 100%).

1.3 Versuchsablauf

Das Gewebe wurde etwa 15 Minuten lang in das zuvor beschriebene Medium mit einer Keimkonzentration > $10^6$ Keime/ml eingebracht. Dann wurde das Gewebe 1 bis 3 Minuten lang in 10 bis 20°C kaltes Wasser eingelegt (keine Spülung). Danach wurde das Gewebe aus dem Wasser genommen und in eine keimfreie Nährlösung eingebracht, die dann inkubiert und bebrütet wurde. Nach 24 bis 72 Stunden wurde die Keimkonzentration in der Nährlösung bestimmt. Die ermittelten Werte wurden auf die primäre Keimmenge bezogen, wobei die folgenden Ergebnisse erhalten wurden:

|  | I. | II. | III. |
|---|---|---|---|
| - E. coli | 15% | 12% | 9% |
| - Staph. aureus | 18 % | 16 % | 16 % |
| - Strep. pyogenes | 5 % | 6 % | 4 % |
| - Enterokokken | 7% | 5 % | 8% |
| - Strept. bovis | 17% | 12% | 14% |
| - Pseud. pyogenes | 6 % | 4 % | 3 % |

Die gemessenen Keimdichten/Konzentrationen nach erneuter Inkubation zeigen bei allen Keimspezies hochsignifikant erniedrigte Werte.

Beispiel 2: Keimreduzierende Eigenschaften des erfindungsgemäßen Gewebes bei der Reinigung von Eutern von Milchkühen

Die Untersuchungen wurden in 3 Milcherzeugungsbetrieben bei je 5 Kühen insgesamt während 10 Melkzeiten durchgeführt. Die eine Euterhälfte wurde mit dem im Betrieb üblichen Reinigungstuch mit desinfektionsmittelgetränktem Wasser gereinigt, die andere Hälfte des Euters mit dem erfindungsgemäßen Gewebe. Die Euterhälften wurden bei jeder Melkzeit gewechselt. Nach der Reinigung wurde das erfindungsgemäße Gewebe in Wasser ohne Reinigungs- und Desinfektionsmittel gelegt. Die Tücher der Vergleichsgruppe wurden ebenfalls in Wasser gelegt, wobei dem Wasser in einem Betrieb zusätzlich noch ein Desinfektionsmittel zugesetzt worden war. Von dem erfindungsgemäßen Gewebe und dem Vergleichstuch wurden vor und nach der Euterreinigung auf Ass. Agar (Antibiotika-Sulfonamid-Sensibilitäts-Agar) eine Abklatschkultur gemacht und bei 38 bis 40°C 18 Stunden lang gebrütet.

Ergebnisse:

Bei der Keimdifferenzierung mittels Selektivnährböden wurden in erster Linie folgende Keime gefunden:

- E. coli
- Streptokokken
- Staphylokokken
- Enterokokken

Setzt man die primäre Bakterienmenge gleich 100%, ergaben sich folgende Werte:

| | Erfindungsgemäßes Gewebe | Betriebseigenes Reinigungstuch | |
|---|---|---|---|
| | | ohne Desinfektion | mit Desinfektion |
| E. coli | 10% | 92% | 35% |
| Streptokokken | 3% | 87% | 25% |

(fortgesetzt)

| | Erfindungsgemäßes Gewebe | Betriebseigenes Reinigungstuch | |
|---|---|---|---|
| | | ohne Desinfektion | mit Desinfektion |
| Staphylokokken | 2% | 78% | 20% |
| Enterokokken | 9% | 83% | 41% |

Die statistische Auswertung der Ergebnisse zeigt eindeutig die hohe keimverhindernde Eigenschaft des erfindungsgemäßen Gewebes. Dadurch wird die Gefahr der Übertragung von Krankheitserregern von einer Kuh auf die andere beim Melken deutlich reduziert.

Beispiel 3: Reinigung eines kontaminierten erfindungsgemäßen Gewebes - Vergleich mit einem herkömmlichen Baumwollgewebe

Das erfindungsgemäße Gewebe wurde in etwa 6 x 6 cm große Stücke geschnitten, die zum Abwischen von kontaminierten Flächen benutzt wurden. Als Vergleichsmaterial diente mehrfach gewaschenes Baumwollgewebe, aus dem Lappen von ungefähr gleichem Gewicht (siehe Tabellen 2 bis 6) geschnitten wurden. Als Versuchsflächen dienten glasierte Keramikfliesen (5 x 5 cm), welche wahlweise mit den Versuchskeimen Enterococcus faecium, Escherichia coli und Staphylococcus aureus beimpft wurden. Die Fliesenstücke wurden mit 50 µl einer Übernachtkultur der Testkeime beimpft, so daß die Zahl der von den Fliesen wieder zu gewinnenden Keime zwischen $3,4 \times 10^5$ und $8,9 \times 10^6$ lag. Der Grad der Kontamination der Testflächen wurde durch Keimzahlbestimmung in der Impfsuspension einerseits und Bestimmung auf den beimpften Flächen durch Abspülen nach dem Antrocknen andererseits kontrolliert. Das Antrocknen erfolgte durch 20-minütige Exposition in einer Laminar Flow Werkbank bei ca. 25°C. Danach wurden die Flächen mit den Versuchsgeweben abgewischt und auf den Fliesen durch Abspülen in 100 ml sterilem, destilliertem Wasser und Ausspateln von Anteilen der Spüllösung auf Nähragarplatten die Keimzahl bestimmt. In den Versuchsgeweben wurde die von den Fliesen aufgenommene Keimzahl ebenfalls durch Ausspülen bei Zimmertemperatur in 200 ml destilliertem Wasser, welches durch Ausdrücken mit der Hand unterstützt wurde, und Verimpfen der Spülflüssigkeit auf Nähragarplatten, bestimmt. Die in den Versuchsgeweben nach dem Ausspülen noch verbliebene Keimmenge wurde durch ein zweites Ausspülen und in der letzten Versuchsreihe durch ein drittes Ausspülen ermittelt. In den folgenden Tabellen sind daher Keimzahlen aus folgenden Suspensionen angegeben:

Übernachtkultur (Impfsuspension)
Spülflüssigkeit der beimpften Fläche
Spülflüssigkeit der abgewischten Fläche
Spülflüssigkeit aus dem Gewebe nach dem Abwischen der Fliesen
Spülflüssigkeit aus dem Gewebe nach dem ersten Ausspülen
Spülflüssigkeit aus dem Gewebe nach dem zweiten Ausspülen

Die Versuchsergebnisse sind in den folgenden Tabellen zusammengefaßt: Tabelle 2: E. coli ATCC 11229, Tabelle 3: Enterococcus faecium, Tabelle 4: ebenfalls Enterococcus faecium, Tabelle 5: Staphylococcus aureus ATCC 6538 und Tabelle 6: Versuche mit allen drei Testkeimen. Die Versuchsdurchführung war für die Tabellen 2 bis 5 im wesentlichen identisch. Tabelle 3 und 4 (beide mit Enterococcus faecium) unterscheiden sich durch eine geringere Einsaat auf den beimpften Flächen. Bei Tabelle 6 wurde zusätzlich das Versuchsgewebe und das Kontrollgewebe ein zusätzliches Mal ausgespült um festzustellen, ob sich ein Unterschied im Keimgehalt des Gewebes nach einmaligem Ausspülen ergibt.

## TABELLE 2

Keim: E. coli ATCC 11229

| Beimpfung mit Übernachtkultur KBE/50 µl | I. E. coli $2,6 \times 10^6$ auf Fliese | | II. E. coli $1,4 \times 10^7$ auf Fliese | | III. E. coli $3,0 \times 10^7$ auf Fliese | |
|---|---|---|---|---|---|---|
| Keimzahlbestimmungen | erfindungsge-mäßes Gewebe | Baumwolle | erfindungsge-mäßes Gewebe | Baumwolle | erfindungsge-mäßes Gewebe | Baumwolle |
| - auf der beimpften Fläche | $5,6 \times 10^5$ | $5,6 \times 10^5$ | $3,7 \times 10^5$ | $3,7 \times 10^5$ | $3,4 \times 10^5$ | $3,4 \times 10^5$ |
| - im Gewebe nach Abwischen der Fliese | $4,8 \times 10^5$ | $4,0 \times 10^5$ | $3,8 \times 10^5$ | $3,0 \times 10^5$ | $5,8 \times 10^5$ | $1,8 \times 10^5$ |
| - im Gewebe nach Ausspülen | $7,0 \times 10^3$ | $6,0 \times 10^3$ | $3,0 \times 10^3$ | $6,0 \times 10^3$ | $5,0 \times 10^3$ | $4,0 \times 10^3$ |
| - auf der Fliese nach dem Abwischen | $1,2 \times 10^4$ | $2,3 \times 10^4$ | 0,0 | $6,5 \times 10^3$ | $3,6 \times 10^4$ | $6,0 \times 10^3$ |
| Gewicht 6 x 6 cm in g | 1,941 | | 1,998 | 0,875 | 2,106 | 1,951 |

EP 1 012 359 B1

## TABELLE 3

Keim: Enterococcus faecium

| Beimpfung mit Übernachtkultur KBE/50 µl | I. $2,0 \times 10^7$ auf Fliese | | II. $2,1 \times 10^7$ auf Fliese | | |
|---|---|---|---|---|---|
| Keimzahlbestimmungen | erfindungsge-mäßes Gewebe | Baumwolle | erfindungsge-mäßes Gewebe | erfindungsge-mäßes Gewebe | Baumwolle |
| - auf der beimpften Fläche | $4,8 \times 10^5$ | $4,8 \times 10^5$ | $6,0 \times 10^5$ | $6,0 \times 10^5$ | $6,0 \times 10^5$ |
| - im Gewebe nach Abwischen der Fliese | $3,6 \times 10^5$ | $3,2 \times 10^5$ | $5,0 \times 10^5$ | $4,0 \times 10^5$ | $1,8 \times 10^5$ |
| - im Gewebe nach Ausspülen | $2,0 \times 10^3$ | $5,0 \times 10^3$ | $4,0 \times 10^3$ | $3,0 \times 10^3$ | $2,0 \times 10^3$ |
| - auf der Fliese nach dem Abwischen | $3,0 \times 10^3$ | $1,0 \times 10^4$ | $5,0 \times 10^3$ | $4,0 \times 10^3$ | $3,0 \times 10^3$ |
| Gewicht 6 x 6 cm in g | 1,679 | 1,017 | 1,852 | 1,784 | 0,950 |
| Vorverkeimung des Lappens | $1,0 \times 10^3$ | | $3,0 \times 10^3$ | $3,0 \times 10^3$ | |

## TABELLE 4

Keim: Enterococcus faecium

| Beimpfung mit Übernachtkultur KBE/50 µl | III. $1,8 \times 10^7$ auf Fliese | | | IV. $3,0 \times 10^7$ auf Fliese | |
|---|---|---|---|---|---|
| Keimzahlbestimmungen | erfindungsge-mäßes Gewebe | erfindungsge-mäßes Gewebe | Baumwolle | erfindungsge-mäßes Gewebe | Baumwolle |
| - auf der beimpften Fläche | $8,9 \times 10^6$ | $4,9 \times 10^5$ | $8,9 \times 10^6$ | $6,7 \times 10^5$ | $6,7 \times 10^5$ |
| - im Gewebe nach Abwischen der Fliese | $6,2 \times 10^6$ | $2,6 \times 10^5$ | $4,8 \times 10^5$ | $6,0 \times 10^5$ | $4,6 \times 10^5$ |
| - im Gewebe nach Ausspülen | $7,9 \times 10^4$ | $5,0 \times 10^3$ | $3,6 \times 10^4$ | $2,0 \times 10^3$ | $6,0 \times 10^3$ |
| - auf der Fliese nach dem Abwischen | $7,1 \times 10^4$ | $5,0 \times 10^3$ | $5,6 \times 10^4$ | $7,5 \times 10^3$ | $5,5 \times 10^3$ |
| Gewicht 6 x 6 cm in g | 1,948 | 1,765 | 0,775 | 2,059 | 1,968 |
| Vorverkeimung des Lappens | $2,0 \times 10^3$ | $4,0 \times 10^3$ | | | |

EP 1 012 359 B1

## TABELLE 5

Keim: Staphylococcus aureus ATCC 6538

| Beimpfung mit Übernachtkultur KBE/50 µl | I. Staph. aureus $7,5 \times 10^7$ auf Fliese | | II. Staph. aureus $4,0 \times 10^7$ auf Fliese | | III. Staph. aureus $6,0 \times 10^7$ auf Fliese | |
|---|---|---|---|---|---|---|
| Keimzahlbestimmungen | erfindungsge-mäßes Gewebe | Baumwolle | erfindungsge-mäßes Gewebe | Baumwolle | erfindungsge-mäßes Gewebe | Baumwolle |
| - auf der beimpften Fläche | $4,8 \times 10^6$ | $4,8 \times 10^6$ | $2,3 \times 10^6$ | $2,3 \times 10^6$ | $7,0 \times 10^6$ | $7,0 \times 10^6$ |
| - im Gewebe nach Abwischen der Fliese | $4,6 \times 10^6$ | $1,1 \times 10^6$ | $1,6 \times 10^6$ | $1,0 \times 10^5$ | $7,0 \times 10^6$ | $5,0 \times 10^6$ |
| - im Gewebe nach Ausspülen | $6,6 \times 10^4$ | $5,0 \times 10^2$ | $1,6 \times 10^4$ | 0,0 | $9,0 \times 10^4$ | $1,3 \times 10^5$ |
| - auf der Fliese nach dem Abwischen | $1,9 \times 10^5$ | $6,0 \times 10^4$ | $6,4 \times 10^4$ | $1,4 \times 10^4$ | $7,4 \times 10^4$ | $7,5 \times 10^4$ |
| Gewicht 6 x 6 cm in g | 1,987 | | 1,855 | 0,960 | 1,939 | 1,937 |

EP 1 012 359 B1

EP 1 012 359 B1

TABELLE 6

| Beimpfung mit Übernachtkultur KBE/50 µl | E. faecium $3,9 \times 10^7$ auf Fliese | | E. coli $3,8 \times 10^7$ auf Fliese | | Staph. aureus $6,0 \times 10^7$ auf Fliese | |
|---|---|---|---|---|---|---|
| Keimzahlbestimmungen | erfindungsge-mäßes Gewebe | Baumwolle | erfindungsge-mäßes Gewebe | Baumwolle | erfindungsge-mäßes Gewebe | Baumwolle |
| - auf der beimpften Fläche | $1,0 \times 10^6$ | $1,0 \times 10^6$ | $3,8 \times 10^5$ | $3,8 \times 10^5$ | $6,8 \times 10^6$ | $6,8 \times 10^6$ |
| - im Gewebe nach Abwischen der Fliese | $4,6 \times 10^5$ | $5,0 \times 10^5$ | $3,8 \times 10^5$ | $4,4 \times 10^5$ | $5,0 \times 10^6$ | $2,6 \times 10^6$ |
| - im Gewebe nach 1 x Ausspülen | $8,0 \times 10^3$ | $1,0 \times 10^4$ | $7,2 \times 10^3$ | $8,8 \times 10^3$ | $4,6 \times 10^4$ | $1,4 \times 10^5$ |
| - auf der Fliese nach dem Abwischen | $6,0 \times 10^2$ | $3,3 \times 10^3$ | $1,5 \times 10^3$ | $5,2 \times 10^3$ | $7,8 \times 10^4$ | $1,0 \times 10^5$ |
| - im Lappen nach 2 x Abspülen | $6,0 \times 10^1$ | $8,4 \times 10^2$ | $2,0 \times 10^2$ | $6,8 \times 10^2$ | $6,0 \times 10^2$ | $4,4 \times 10^3$ |
| Gewicht 6 x 6 cm in g | 1,933 | 2,0 | 2,034 | 2,0 | 2,015 | 2,0 |

Die quantitative Auswertung der Versuchsergebnisse zeigt, daß die Fliesen ungefähr im gleichen Ausmaß vom erfindungsgemäßen Gewebe und vom Baumwollgewebe gereinigt werden. Andeutungsweise ergibt sich ein besserer Reinigungseffekt durch das erfindungsgemäße Gewebe. Ein deutlicher Unterschied zeigt sich bei der Keimzahlbestimmung in den Wischgeweben nach dem Gebrauch. Zu diesem Zeitpunkt nach dem Abwischen der Fliesen verbleibt im erfindungsgemäßen Gewebe eine größere Anzahl der Mikroorganismen als im Baumwollgewebe. Nach dem ersten Ausspülen sind die Keimzahlen in den beiden Geweben in etwa gleich, nach dem zweiten Ausspülen (Tabelle 6) liegen die Keimzahlen im erfindungsgemäßen Gewebe bei allen drei Ansätzen unter denen im Baumwollgewebe.

Die Ergebnisse zeigen, daß das erfindungsgemäße Gewebe durch einfaches Ausspülen in zusatzstofffreiem Wasser besser von den aufgenommenen Keimen befreit werden kann als konventionelles Baumwollgewebe und bei der Reinigung von Oberflächen dem Baumwollgewebe überlegen ist. Das erfindungsgemäße Gewebe ist daher zur Reinigung von Oberflächen und andere Verwendungen, bei denen es auf eine reversible Aufnahme von bakterienhaltigem Material ankommt, besonders gut geeignet.

Beispiel 4: Herstellung von Wasser mit reduzierter Oberflächenspannung

Für die Demonstration der Herstellung von Wasser mit reduzierter Oberflächenspannung kann vorzugsweise Trinkwasser mit einem Härtegrad von 5 bis 25 mit beliebigen Ionengemischen verwendet werden; das Verfahren ist in einem Temperaturbereich von bevorzugt 5 bis 30°C, optimalerweise zwischen 15 und 25°C durchführbar und führt zu Wasser mit deutlich reduzierter Oberflächenspannung. Es ist bevorzugt, ein Gefäß mit völlig homogener Oberfläche, z.B. aus Glas, Metall, Emaille oder Keramik, zu verwenden, in dem das Wasser in Kontakt mit dem erfindungsgemäßen Gewebe kommen kann.

Die erfindungsgemäße Faser sollte bevorzugt in ein Gewebe mit einem Faserrasen von 0,5 cm Länge gewoben werden. Unter diesen Voraussetzungen ist bei entsprechender gleichmäßiger Verteilung des Gewebes im Wasser für eine Menge von 0,16 m$^3$ Wasser eine Fläche von 1 m$^2$ Gewebe mit beidseitigem Faserrasen für eine Inkubationsdauer von 5 bis 10 Sekunden erforderlich. Der verwendete Wasserbehälter kann jede beliebige Form besitzen. Es ist möglich, das Gewebe in einem Becken fest zu installieren oder bei Bedarf durch eine entsprechende Mechanik in ein Becken einzutauchen. Der Zustand der reduzierten Oberflächenspannung bleibt nach dem Entfernen des Gewebes aus dem Behältnis bzw. Entfernen des Wassers aus dem Behältnis bei einer Temperatur bis zu 40°C für die Dauer von mindestens 60 Minuten in vollem Umfang aufrechterhalten, nach 120 Minuten tritt ein langsamer Wiederanstieg der Oberflächenspannung auf normale Werte (72 bis 78 mN/m) ein. In diesem Zeitraum ist es möglich, das erzeugte Wasser mit oder auch ohne Faserkontakt in Synthese-, Extraktions- oder Fermentationsprozesse einzubringen. Dabei ist es z.B. auch möglich, zur Verbesserung und Beschleunigung von Fermentationsprozessen beim Brauen das Wasser in alle Fermentationsstufen, auch bei Temperaturen über 40°C, einzubringen.

Ebenso ist es auch möglich, nach Abkühlen der Maische vor Zusetzen der Hefe im Tauchverfahren die Oberflächenspannung entsprechend herabzusetzen, um ein verbessertes Brauergebnis zu erzielen.

Beispiel 4.1:

Es wurden 3 erfindungsgemäße Gewebeproben "S10", "L01" und "L02" untersucht, die sich hinsichtlich der Qualität ihrer Oberflächenstruktur unterscheiden.

Die Messungen wurden in Glasgefäßen durchgeführt, wobei eine wirksame Fläche der Gewebeproben von 400 cm$^2$ in 4 Liter Wasser untersucht wurde. Die Untersuchungstemperatur lag bei 20°C.

Die Messungen erfolgten

    1. ohne vorherige Spülung
    2. nach drei Spülungen
    3. nach sechs Spülungen
    4. nach neun Spülungen

des jeweiligen Gewebes.

Die Vergleichsmessungen erfolgten in bidestilliertem Wasser und ortsüblichem Trinkwasser.

Die Messung der Oberflächenspannung erfolgte in dem in Figur 22 dargestellten Tensiometer.

Versuchsbeschreibung:

Ein 5-Liter-Becher aus Duranglas wird mit 4 Litern ortsüblichem Trinkwasser von 20°C befüllt, wovon eine Probe zur Messung der Oberflächenspannung entnommen wird. Danach wird das zu untersuchende Gewebe eingetaucht, mehrfach durchgeknetet und unter Auswinden wieder entfernt. Von dem zurückbleibenden Wasser werden mehrfach Proben entnommen und die Oberflächenspannung bestimmt. Nun wird das Gewebe in gleicher Weise zweimal ohne anschließende Messung in jeweils 4 Liter frischem Wasser gespült. Der gesamte Vorgang wird dreimal wiederholt.

Die Messung der Oberflächenspannung erfolgt folgendermaßen: Die Wasserprobe wird in das dem Tensiometer zugehörige Probengefäß eingefüllt und dieses in das bewegliche Tischchen eingestellt und hochgefahren, bis der Platinring in die Probe eintaucht. Dann wird der Servomotor aktiviert, der die Wasserprobe nach unten fährt, wobei eine Wasserlamelle am Ring herausgezogen wird. Der Motor stoppt am Spannungsmaximum, wenn der Oberflächenfilm nachgibt und keine Zugkraft mehr auf die Waage, an der der Ring hängt, einwirkt. Der Maximalwert kann nun an der Digitalanzeige abgelesen werden.

Einseitiges Gewebe:

Zunächst wird die Wirkung von einseitigen Gewebeproben der Größe 400 cm$^2$ untersucht. Hierzu wird das jeweilige Gewebe in 4 Liter Trinkwasser bei 20°C gegeben und nach mehrfachem Umrühren wieder entfernt. Dies geschieht zunächst mit ungespülten Geweben. Anschließend wird eine Probe entnommen und die Oberflächenspannung bestimmt.

Im Anschluß daran wird das Gewebe 3-fach gespült und der Versuch wiederholt. Eine Versuchswiederholung erfolgt ebenfalls nach einer 6-fachen bzw. 9-fachen Spülung des geweiligen Gewebes.

In den Figuren 23 bis 25 sind die Oberflächenspannungen nach 0, 3, 6 und 9 Spülungen denen von bidestilliertem Wasser bzw. Trinkwasser gegenübergestellt. Bei allen Spülungen ist ein starker Lufteintrag ins Versuchswasser sowie "Fusseln" zu beobachten, was zu schwankenden Meßwerten führt. Daher wurden mehrere Messungen durchgeführt und jeweils die arithmetischen Mittelwerte angegeben.

Bei den Figuren 23 bis 25 kann tendentiell die gleiche Aussage getroffen werden: Durch das Eintauchen des jeweiligen Gewebes in das Versuchswasser wird die Oberflächenspannung um 30-40% von ca. 71 mN/m auf 40-50 mN/m herabgesetzt. Mit zunehmender Anzahl der Spülungen des jeweiligen Gewebes wird die oberflächenentspannende Wirkung der Gewebe geringer.

Doppelseitiges Gewebe:

Die doppelseitigen Gewebe weisen im Vergleich zu den einseitigen Geweben bei gleicher Fläche von 400 cm$^2$ mehr als die doppelte Masse auf. In Tabelle 7 sind die ermittelten Massen der jeweiligen Gewebe angegeben.

TABELLE 7:

| Masse der untersuchten Gewebe | | |
|---|---|---|
| Typ | einseitig | zweiseitig |
| L01 | 16,10 g | 38,87 g |
| L02 | 16,69 g | 38,44 g |
| S10 | 15,53 g | 41,31 g |

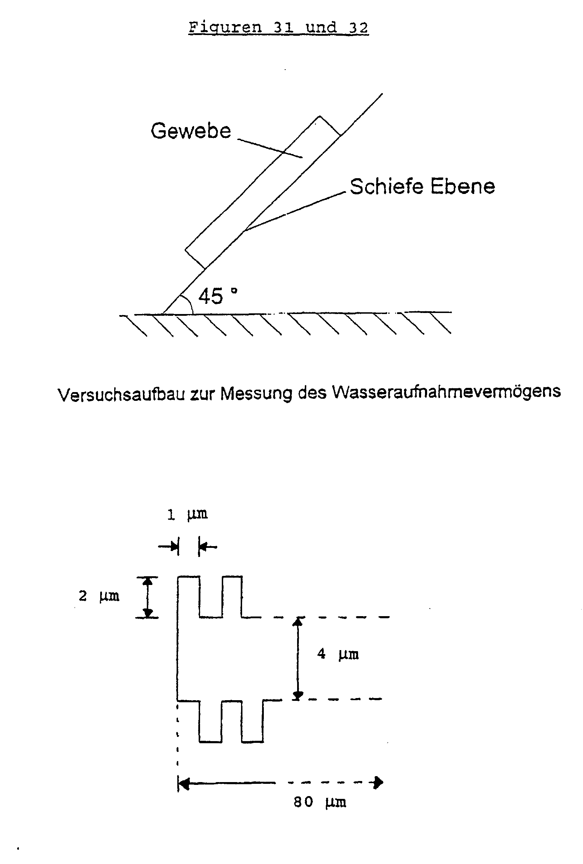

In den Figuren 26 bis 28 ist die oberflächenentspannende Wirkung der doppelseitigen Gewebe in Abhängigkeit der Spülungen angegeben.

Die doppelseitigen Gewebe L01 und S10 reduzieren die Oberflächenspannung der Probe von etwa 72 mN/m auf 60-62 mN/m. Dies entspricht einer Reduzierung um 14-17%. Diese Wirkung ist unabhängig von der Anzahl der Spülungen und bleibt auch nach 9 Spülungen auf diesem Niveau bestehen.

Beim doppelseitigen Gewebe L02 wird die Oberflächenspannung zunächst um 42% von 72 mN/m auf 42 mN/m reduziert. Anschließend nimmt die oberflächenspannungsreduzierende Wirkung jedoch mit zunehmender Anzahl von Spülungen ab und erreicht nach 9 Spülungen einen Wert von 70,4 mN/m.

Oberflächenspannung mit im Wasser verbleibenden Geweben:

In diesem Versuch wurden die jeweiligen Gewebe nicht aus dem Wasser entfernt, bevor die Oberflächenspannung gemessen wurde. Es erfolgten mehrere zeitlich direkt nacheinander folgende Messungen. Der Verlauf der Oberflächenspannung der Gewebe L01, L02 und S10 in Abhängigkeit von der Zeit ist in Figur 29 dargestellt. Ein konstanter Wert pendelte sich erst nach ca. 10 Minuten ein . Die Wassertemperatur betrug konstant 20°C.

Während sich das Wasser mit den Geweben L01 und L02 auf einen Wert von 69 bzw. 68 mN/m einpendelt, weist das Wasser mit Gewebe S10 einen deutlich geringeren Endwert von 62 mN/m auf.

Zusammenfassend wird festgestellt:

Beim primären Kontakt des erfindungsgemäßen Gewebes mit Wasser konnte die Oberflächenspannung von Trinkwasser von etwa 72 mN/m um bis zu 40% auf Werte um 40 mN/m reduziert werden. Dieser Effekt wird jedoch bei den einseitigen Geweben mit zunehmender Anzahl der Spülungen der Gewebe bei Wiederholung des Versuches geringer. Bei den doppelseitigen Geweben L01 und S10 konnte auch nach jeweils 9-facher Spülung des Gewebes noch eine Reduzierung der Oberflächenspannung von Trinkwasser um 14-17% festgestellt werden. Hier pendelte sich ein konstanter Wert ein, der sich nur geringfügig von den Werten nach 3- bzw. 6-facher Spülung des Gewebes unterschied.

Das doppelseitige Gewebe L02 verhielt sich analog den einseitigen Geweben, bei denen die oberflächenentspannende Wirkung mit zunehmender Anzahl der Spülungen der Gewebe sank.

Werden die Gewebe im Wasser belassen, so pendelt sich im günstigsten Fall ein konstanter Wert von 62 mN/m für die Oberflächenspannung nach etwa 10 Minuten ein.

Im Vergleich zu Trinkwasser (72 mN/m) bedeutet dies eine Verminderung der Oberflächenspannung um 14%.

Beispiel 4.2:

In diesem Versuch wurde die oberflächenentspannende Wirkung bei Wasser des in Beispiel 4.1 beschriebenen doppelseitigen Gewebes "L01" nach einer Austrocknungsphase von mehreren Monaten getestet.

Die Messungen wurden in Glasgefäßen durchgeführt. Eine wirksame Fläche von 400 cm$^2$ des Gewebes wurde in 4 Liter Wasser untersucht. Die Messung erfolgte bei 20°C.

Die Vergleichsmessungen erfolgen in bidestilliertem Wasser und ortsüblichem Trinkwasser.

Die Messung der Oberlfächenspannung erfolgte mit dem in Figur 22 dargestellten Tensiometer.

Versuchsbeschreibung:

Ein 5-Liter-Becher aus Duranglas wird mit 4 Litern ortsüblichem Trinkwasser bzw. mit bidestilliertem Wasser mit einer Temperatur von 20°C befüllt, wovon jeweils eine Probe zur Messung der Oberflächenspannung entnommen wird. Danach wird das zu untersuchende Gewebe eingetaucht, mehrfach durchgeknetet und unter Auswinden wieder entfernt. Von dem zurückbleibenden Wasser werden mehrfach Proben entnommen und die Oberflächenspannung bestimmt.

Die Messung der Oberflächenspannung erfolgt folgendermaßen: Die Wasserprobe wird in das dem Tensiometer zugehörige Probengefäß eingefüllt und dieses in das bewegliche Tischchen eingestellt und hochgefahren, bis der Platinring in die Probe eintaucht. Dann wird der Servomotor aktiviert, der die Wasserprobe nach unten fährt, wobei eine Wasserlamelle am Ring herausgezogen wird. Der Motor stoppt am Spannungsmaximum, wenn der Oberflächenfilm nachgibt und keine Zugkraft mehr auf die Waage, an der der Ring hängt, einwirkt. Der Maximalwert kann nun an der Digitalanzeige abgelesen werden.

In Figur 30 ist die Oberflächenspannung von Wasser nach dem Kontakt mit dem nach der Trocknungsphase untersuchten Gewebe "L01" derjenigen desselben Gewebes vor der Trocknungsphase bzw. denjenigen von ortsüblichem Trinkwasser bzw. bidestilliertem Wasser gegenübergestellt. Bei der Messung war ein starker Lufteintrag ins Versuchswasser zu beobachten, was zu schwankenden Meßwerten führte. Daher wurden mehrere Messungen durchgeführt und der arithmetische Mittelwert angegeben. Die Einzelmessungen sind in Tabelle 8 angegeben.

TABELLE 8:

| Oberflächenentspannende Wirkung des erfindungsgemäßen Gewebes "L01" vor bzw. nach der Trocknungsphase (Einzelwerte) | | |
|---|---|---|
| Einzelmessung Nr. | Oberflächenspannung (mN/m) | |
| | L01 vor Trocknungsphase | L01 nach Trocknungsphase |
| 1 | 62,0 | 58,2 |
| 2 | 62,4 | 58,4 |
| 3 | 62,5 | 58,3 |
| 4 | 62,1 | 58,4 |

TABELLE 8:   (fortgesetzt)

| Oberflächenentspannende Wirkung des erfindungsgemäßen Gewebes "L01" vor bzw. nach der Trocknungsphase (Einzelwerte) | | |
|---|---|---|
| Einzelmessung Nr. | Oberflächenspannung (mN/m) | |
| | L01 vor Trocknungsphase | L01 nach Trocknungsphase |
| 5 | 62,5 | 58,7 |
| Mittelwert x | 62,3 | 58,4 |

Die oberflächenspannungsreduzierte Wirkung des doppelseitigen Gewebes "L01" hat sich nach der Trocknungsphase nicht verschlechtert. Die Oberflächenspannung konnte von 71,4 mN/m auf 58,4 mN/m, also um 18 %, reduziert werden.

Dieser Versuch zeigt, daß die oberflächenentspannende Wirkung des erfindungsgemäßen Gewebes auf die Faserstruktur, und nicht auf aus dem Gewebe ausgelaugte Stoffe, zurückzuführen ist. Die oberflächenentspannende Wirkung bleibt also auch nach einer längeren Trocknungsperiode erhalten.

Beispiel 4.3:

Die folgenden Tabellen 9 und 10 zeigen Ergebnisse von weiteren durchgeführten Untersuchungen. Die Gewebe wurden vor den Untersuchungen jeweils ein- oder zweimal mit Wasser gespült, um eventuelle Verunreinigungen aus dem Herstellungsprozeß zu entfernen.

TABELLE 9:  Meßergebnisse bei verschiedenen Gefäßen

| Messung Nr. | Gefäß | Menge (l) | Tempe-ratur (°C) | Sonstiges | Warte-zeit (min) | Oberflächenspannung vor Behandlung (mN/m) | Oberflächenspannung nach Behandlung (mN/m) |
|---|---|---|---|---|---|---|---|
| 1 | Glas | 4 | 17 | bidest. Wasser | 0 | 71,9/71,9 | - |
| 2 | Glas | 4 | 17 | Trinkwasser (TW) | 0 | 71,7/71,6 | - |
| 3 | Glas | 4 | 16 | TW + einfach gespültes Gewebe | 0 | 71,6 | 52,4 |
| 4 | Glas | 4 | 15 | TW + zweifach gespültes Gewebe | 0 | 72,2 | 52,5 |
| 5 | Kunststoff | 10 | 14 | TW + zweifach gespültes Gewebe | 0 | - | 65,4 |
| 6 | Glas | 4 | 13 | TW + zweifach gespültes Gewebe | 0 | - | 58,8 |
| 7 | Kunststoff | 4 | 14 | TW + zweifach gespültes Gewebe | 0 | 72,6 | 62,2 |
| 8 | Kunststoff | 10 | 14 | TW + zweifach gespültes Gewebe | 0 | 71,1 | 60,7 |

TABELLE 10: Meßergebnisse bei verschiedenen Temperaturen

| 1 | Glas | 4 | 15 | TW + Gewebe | 0 | 69,7 | 59,9 |
|---|---|---|---|---|---|---|---|
| 2 | Glas | 4 | 20 | TW + Gewebe | 0 | 68,7 | 48,0 |
| 3 | Glas | 4 | 25 | TW + Gewebe | 0 | 66,4 | 46,5 |
| 4 | Glas | 4 | 30 | TW + Gewebe | 0 | 65,5 | 46,5 |
| 5 | Glas | 4 | 35 | TW + Gewebe | 0 | 64,4 | 46,4 |

EP 1 012 359 B1

Bestimmung des Wasseraufnahmevermögens des erfindungsgemäßen Gewebes

Das gewichtsbezogene Wasseraufnahme- bzw. Rückhaltevermögen der erfindungsgemäßen Gewebe wurde untersucht. Das Wasserrückhaltevermögen der Gewebe wurde auf einer 45° geneigten Fläche für 60 Sekunden bei Raumtemperatur und Wassersättigung untersucht. Dabei wurde die in Figur 31 gezeigte Anordnung verwendet.

Versuchsbeschreibung:

Das trockene Gewebe wird zuerst gewogen, anschließend 20 Minuten in ausreichender Wassermenge eingeweicht. Dann wird das tropfnasse Gewebe 60 Sekunden auf eine schiefe Ebene (Figur 31) gelegt und danach sofort gewogen. Das Wasseraufnahmevermögen ergibt sich aus der Differenz der Masse des jeweils vollgesogenen Gewebes und der Trockenmasse des Gewebes. Dieser Versuch wird ca. 8 mal wiederholt, wobei das Gewebe zwischen den Versuchen jeweils 2 Minuten zum Vollsaugen wieder in das Wasser gelegt wird. Aus den Werten der Einzelversuche wird der arithmetische Mittelwert ermittelt. Der Versuch wird bei Raumtemperatur (ca. 22°C) durchgeführt.

Die folgende Tabelle 11 zeigt das Wasserrückhaltevermögen der Gewebe. Die Gewebe wurden jeweils vor Beginn der Untersuchungen 20 Minuten eingeweicht. Zwischen den Messungen erfolgte eine Pause von jeweils 2 Minuten.

TABELLE 11:

| Wasserrückhaltevermögen der Gewebe | | | |
|---|---|---|---|
| Gewebe | Trockengewicht (g) | Abtropfgewicht (g) | Wassermasse (g) | Wasserrückhaltevermögen (x-faches vom Eigengewicht) |
| L01 einseitig | 16,1 | 84,04 | 67,94 | 4,22 |
| L02 einseitig | 16,69 | 110,04 | 93,35 | 5,59 |
| S10 einseitig | 15,53 | 99,72 | 84,19 | 5,42 |
| L01 zweiseitig | 35,87 | 188,08 | 152,21 | 4,24 |
| L02 zweiseitig | 38,44 | 267,81 | 229,37 | 5,97 |
| S10 zweiseitig | 41,31 | 207,88 | 166,57 | 4,03 |

Die vom jeweiligen Gewebe zurückgehaltene Wassermenge wurde aus der Differenz von Abtropfmasse und Trockenmasse ermittelt. Die Tabelle 11 zeigt, daß die jeweiligen Gewebe zwischen dem 4- bis 6-fachen ihrer Trockenmasse an Wasser zurückhalten können.

Modell zur Berechnung der spezifischen Oberfläche des erfindungsgemäßen Gewebes

Für die Berechnung wurden beispielhaft folgende Werte angenommen (siehe Figur 32):

| | |
|---|---|
| Filamentbreite | 80 µm |
| Filamentgrundstrukturhöhe | 4 µm |
| Lamellenhöhe | 2 µm |
| Lamellenbreite | 1 µm |

Daraus ergeben sich

1. Querschnittslinienlänge eines Filamentes

$$2 \times 40 \times 6 \ \mu m + 2 \times 4 \ \mu m = 488 \ \mu m$$

2. produktionsbedingte Filamentlänge: 0,5 cm
Oberfläche eines Einzelfilamentes:

$$488 \ \mu m \times 0,5 \ cm = 244 \times 10^{-4} \ cm^2$$

Eine Faser enthält als Produktionsmerkmal 80 Filamente, die durchschnittliche Faserzahl pro Quadratmillimeter beträgt 9 Fasern.

Daraus ergibt sich eine spezifische Oberfläche pro Quadratzentimeter von $72000 \times 244 \times 10^{-4} cm^2 = 1756,8$ $cm^2$ Oberfläche pro Quadratzentimeter Gewebefläche.

Ein Tuch aus doppelseitigem Gewebe von 20 cm x 23 cm ($460 cm^2$) besitzt somit eine Oberfläche von 161,62 $m^2$.

**Patentansprüche**

1. Verfahren zum Herstellen einer Cellulosefaser aus Hydratcellulose, das die folgenden Schritte umfaßt:

   a) das Behandeln von Zellstoff aus höchstens 1-jährigen Schößlingen von Lauboder Nadelhölzern mit einer Alkalimetallhydroxidlösung, um eine Alkalicellulose zu erhalten,

   b) das Abpressen der überschüssigen Alkalimetallhydroxidlösung aus der erhaltenen Alkalicellulose,

   c) das Zerfasern der Alkalicellulose,

   d) das Vorreifen der zerfaserten Alkalicellulose bis zu einer Reife von 5° bis 30° Hottenroth,

   e) das Behandeln der vorgereiften Alkalicellulose nach dem Naßsulfidverfahren, um die Cellulose zu xanthogenieren,

   f) das Spülen und Verdünnen der xanthogenierten Cellulose mit Wasser, um eine Spinnlösung herzustellen,

   g) das Nachreifen der gespülten und verdünnten Cellulose bis zu einer Reife von 5° bis 30° Hottenroth,

   h) das Filtrieren und anschließende Entlüften der Spinnlösung,

   i) das Einpressen der Spinnlösung in ein Fällbad unter Verwendung von Spinndüsen,

   j) das Abziehen der koagulierenden Fasern unter gleichzeitigem Verdrillen der Fasern, um eine verdrillte Faser herzustellen,

   k) das Entwässern der verdrillten Faser,

   l) das Entschwefeln der verdrillten Faser,

   m) das Waschen der verdrillten Faser mit Wasser,

   n) das Vorentwässern der verdrillten Faser und

   o) das Trocknen der verdrillten Faser.

2. Verfahren nach Anspruch 1, **dadurch gekennzeichnet, daß** der Zellstoff aus höchstens 1-jährigen Schößlingen von Robiniengehölz, Teakholz, Bongassiholz und Bambus ausgewählt ist.

3. Verfahren nach Anspruch 1, **dadurch gekennzeichnet, daß** der Ligningehalt der höchstens 1-jährigen Schößlinge nicht mehr als 7%, bevorzugt nicht mehr als 5% und besonders bevorzugt nicht mehr als 2% beträgt.

4. Verfahren nach Anspruch 1, **dadurch gekennzeichnet, daß** die zur Behandlung des Zellstoffes in Schritt a) verwendete Alkalimetallhydroxidlösung eine Natronlauge mit einem Gehalt an Natriumhydroxid im Bereich von 150 bis 350 g/l ist.

5. Verfahren nach Anspruch 4, **dadurch gekennzeichnet, daß** die Natronlauge etwa 300 g/l Natriumhydroxid enthält.

6. Verfahren nach Anspruch 1, **dadurch gekennzeichnet, daß** die Behandlung des Zellstoffes in Schritt a) bei einer Temperatur im Bereich von 15°C bis 25°C durchgeführt wird.

7. Verfahren nach Anspruch 1, **dadurch gekennzeichnet, daß** das Zerfasern der Alkalicellulose in Schritt c) einen Grobzerkleinerungsschritt und einen Feinzerkleinerungsschritt umfaßt.

8. Verfahren nach Anspruch 1, **dadurch gekennzeichnet, daß** die zerfaserte Alkalicellulose in Schritt d) bei einer Temperatur im Bereich von 60°C bis 80°C vorgereift wird.

9. Verfahren nach Anspruch 8, **dadurch gekennzeichnet, daß** die zerfaserte Alkalicellulose bei einer Temperatur im Bereich von 65°C bis 75°C vorgereift wird.

10. Verfahren nach Anspruch 9, **dadurch gekennzeichnet, daß** die zerfaserte Alkalicellulose bei einer Temperatur von etwa 72°C vorgereift wird.

11. Verfahren nach Anspruch 1, **dadurch gekennzeichnet, daß** die zerfaserte Alkalicellulose in Schritt d) bis zu einer Reife von 8° bis 12° Hottenroth vorgereift wird.

12. Verfahren nach Anspruch 11, **dadurch gekennzeichnet, daß** die zerfaserte Alkalicellulose bis zu einer Reife von etwa 10° Hottenroth vorgereift wird.

13. Verfahren nach Anspruch 1, **dadurch gekennzeichnet, daß** das Naßsulfidverfahren in Schritt e) in einer Lösung mit Schwefelkohlenstoff, Natronlauge und Berol durchgeführt wird.

14. Verfahren nach Anspruch 13, **dadurch gekennzeichnet, daß** der Gehalt an Schwefelkohlenstoff 150 bis 250 g/l und der Gehalt an Natriumhydroxid 250 bis 350 g/l beträgt.

15. Verfahren nach Anspruch 14, **dadurch gekennzeichnet, daß** der Gehalt an Schwefelkohlenstoff 180 bis 210 g/l und der Gehalt an Natriumhydroxid 280 bis 320 g/l beträgt.

16. Verfahren nach Anspruch 1, **dadurch gekennzeichnet, daß** das Nachreifen der Cellulose in Schritt g) bis zu einer Reife von 8° bis 12° Hottenroth durchgeführt wird.

17. Verfahren nach Anspruch 1, **dadurch gekennzeichnet, daß** die Spinnlösung nach dem Nachreifen der Cellulose und vor dem Filtrieren der Spinnlösung mit mindestens einer weiteren Spinnlösung vermischt wird, die mit einem Verfahren hergestellt wurde, das die Schritte a) bis g) gemäß Anspruch 1 umfaßt.

18. Verfahren nach Anspruch 1, **dadurch gekennzeichnet, daß** die Temperatur des Fällbades in Schritt i) 35°C bis 45°C beträgt.

19. Verfahren nach Anspruch 18, **dadurch gekennzeichnet, daß** die Temperatur des Fällbades etwa 40°C beträgt.

20. Verfahren nach Anspruch 1, **dadurch gekennzeichnet, daß** das Fällbad in Schritt i) 70 bis 160 g/l, bevorzugt 90 bis 140 g/l und besonders bevorzugt etwa 120 g/l Schwefelsäure enthält.

21. Verfahren nach Anspruch 1, **dadurch gekennzeichnet, daß** das Fällbad in Schritt i) 0,3 bis 4 g/l, bevorzugt 0,5 bis 2 g/l und besonders bevorzugt etwa 1 g/l Zinksulfat enthält.

22. Verfahren nach Anspruch 1, **dadurch gekennzeichnet, daß** das Fällbad in Schritt i) 0,05 bis 1 g/l, bevorzugt 0,1 bis 0,7 g/l und besonders bevorzugt etwa 0,4 g/l Berol enthält.

23. Verfahren nach Anspruch 1, **dadurch gekennzeichnet, daß** die Spinndüsen in Schritt i) durch Beheizen auf einer Temperatur im Bereich von 55°C bis 75°C gehalten werden.

24. Verfahren nach Anspruch 23, **dadurch gekennzeichnet, daß** die Spinndüsen auf einer Temperatur von 65°C bis 70°C, bevorzugt etwa 67°C gehalten werden.

25. Verfahren nach Anspruch 1, **dadurch gekennzeichnet, daß** die Spinndüsen in Schritt i) oval bis langgestreckt schlitzförmig sind.

EP 1 012 359 B1

**26.** Verfahren nach Anspruch 1, **dadurch gekennzeichnet, daß** das Entwässern der Faser in Schritt k) mit einer Schwefelsäurelösung durchgeführt wird, die bis zu 15 g/l Schwefelsäure enthält.

**27.** Verfahren nach Anspruch 26, **dadurch gekennzeichnet, daß** die zum Entwässern der Faser verwendete Schwefelsäurelösung bis zu 10 g/l Schwefelsäure enthält.

**28.** Verfahren nach Anspruch 1, **dadurch gekennzeichnet, daß** das Entschwefeln der Faser in Schritt l) mit einer Natriumsulfatlösung durchgeführt wird, die 2 bis 5 g/l Natriumsulfat enthält.

**29.** Verfahren nach Anspruch 28, **dadurch gekennzeichnet, daß** die zur Entschwefelung der Faser verwendete Lösung etwa 3 g/l Natriumsulfat enthält.

**30.** Verfahren nach Anspruch 1, **dadurch gekennzeichnet, daß** die verdrillte Faser nach dem Waschen mit Wasser und vor dem Entwässern mit Titandioxid präpariert wird.

**31.** Verfahren nach Anspruch 1, **dadurch gekennzeichnet, daß** das Vorentwässern der Faser in Schritt n) mit Druckluft durchgeführt wird.

**32.** Verfahren nach Anspruch 1, **dadurch gekennzeichnet, daß** das Trocknen der Faser in Schritt o) unter Verwendung von Kanaltrocknern durchgeführt wird.

**33.** Cellulosefaser, erhältlich mit einem Verfahren nach einem der Ansprüche 1 bis 32, **dadurch gekennzeichnet, daß** sie eine Mikrostruktur mit faserparallelen Lamellen aufweist, im wesentlichen kein Lignin enthält und weitgehend frei von Schwefelsäure und Schwefelkohlenstoff ist.

**34.** Cellulosefaser nach Anspruch 33, **dadurch gekennzeichnet, daß** die faserparallelen Lamellen einen Abstand von 1 nm bis 5 μm haben.

**35.** Cellulosefaser nach Anspruch 34, **dadurch gekennzeichnet, daß** die faser-parallelen Lamellen einen Abstand von 200 nm bis 1 μm haben.

**36.** Gewebe, umfassend ein Grundgewebe und einen darin eingewobenen Flor, der die Faser nach einem der Ansprüche 33 bis 35 enthält.

**37.** Gewebe nach Anspruch 36, **dadurch gekennzeichnet, daß** das Grundgewebe eine Gitterstruktur besitzt.

**38.** Gewebe nach Anspruch 36, **dadurch gekennzeichnet, daß** der Flor einen Faserrasen von etwa 0,5 cm Länge zum Grundgewebe bildet.

**39.** Gewebe nach Anspruch 36, **dadurch gekennzeichnet, daß** das Grundgewebe Viskose-Spinnfasern enthält.

**40.** Gewebe nach Anspruch 39, **dadurch gekennzeichnet, daß** das Grundgewebe aus Viskose-Spinnfasern besteht.

**41.** Gewebe nach Anspruch 36, **dadurch gekennzeichnet, daß** der Flor ovale und bändchenförmige Fasern umfaßt.

**42.** Gewebe nach Anspruch 41, **dadurch gekennzeichnet, daß** der Flor aus 50% ovalen und 50% bändchenförmigen Fasern besteht.

**43.** Gewebe nach Anspruch 42, **dadurch gekennzeichnet, daß** der Flor aus je 50% Fasern der Dimension 330 dtex F 60 oval und 330 dtex F 80 bändchenförmig besteht.

**44.** Verwendung des Gewebes nach einem der Ansprüche 36 bis 43 zum Reinigen und Dekontaminieren.

**45.** Verwendung des Gewebes nach einem der Ansprüche 36 bis 43 zum Verringern der Oberflächenspannung von Wasser.

**46.** Verwendung des Gewebes nach einem der Ansprüche 36 bis 43 zur Herstellung von Textilien.

22

**EP 1 012 359 B1**

**47.** Verwendung des Gewebes nach einem der Ansprüche 36 bis 43 zur Herstellung von Bekleidungsmaterialien.

**48.** Verwendung des Gewebes nach einem der Ansprüche 36 bis 43 zur Herstellung von Hygieneartikeln.

**49.** Verwendung des Gewebes nach einem der Ansprüche 36 bis 43 als Partikelfilter.

**50.** Verwendung des Gewebes nach einem der Ansprüche 36 bis 43 als Kondensationskatalysator.

**51.** Verwendung des Gewebes nach einem der Ansprüche 36 bis 43 als Bodenbelag.

**52.** Verwendung des Gewebes nach einem der Ansprüche 36 bis 43 als Bezugsmaterial.

**Claims**

**1.** Process to manufacture a cellulose fibre from hydrate cellulose which comprises the following steps:

a) Treatment of wood pulp derived from shoots no older than 1 year of deciduous trees or conifers with an alkali metal hydroxide solution in order to obtain an alkali cellulose;

b) pressing out of the superfluous alkali metal hydroxide solution from the obtained alkali cellulose;

c) shredding of the alkali cellulose into crumbs;

d) ripening of the alkali cellulose crumbs to a maturity of between 5° and 30° Hottenroth;

e) application of the wet sulphide process to treat the ripened crumbs in order to sulphidise the cellulose;

f) rinsing and diluting of the sulphidised cellulose with water in order to obtain a spinning solution;

g) subsequent ripening of the rinsed and diluted cellulose to a maturity of between 5° and 30° Hottenroth;

h) filtering and downstream deaeration of the spinning solution;

i) injection of the spinning solution into a regenerating bath under application of spinnerets;

j) stripping off the coagulating fibres with simultaneous twisting in order to obtain twisted fibres;

k) dehydrating of the twisted fibres;

l) desulphurisation of the twisted fibres;

m) washing of the twisted fibres with water;

n) predehydrating of the twisted fibres; and

o) drying of the twisted fibres.

**2.** Process in accordance with Claim 1, **characterised in that** the wood pulp derives from shoots no older than 1 year of false acacia trees, teak trees, bongassi trees or bamboo.

**3.** Process in accordance with Claim 1, **characterised in that** the lignin content of the less-than-one-year-old shoots used does not exceed 7%, preferred is no more than 5%, and particularly favourable is no more than 2%.

**4.** Process in accordance with Claim 1, **characterised in that** the alkali metal hydroxide solution used to treat the wood pulp in Step a) is a sodium hydroxide solution which contains between 150 and 350 g/l of sodium hydroxide.

**5.** Process in accordance with Claim 4, **characterised in that** the sodium hydroxide solution contains approx. 300

23

EP 1 012 359 B1

g/l of sodium hydroxide.

6. Process in accordance with Claim 1, **characterised in that** treatment of the wood pulp in Step a) is carried. out at a temperature ranging between 15°C and 25°C.

7. Process in accordance with Claim 1, **characterised in that** the shredding process of the alkali cellulose in Step c) comprises a coarse comminution step and a fine comminution step.

8. Process in accordance with Claim 1, **characterised in that** the alkali cellulose crumbs in Step d) are ripened at a temperature ranging between 60°C and 80°C.

9. Process in accordance with Claim 8, **characterised in that** the alkali cellulose crumbs are ripened at a temperature of between 65°C and 75°C.

10. Process in accordance with Claim 9, **characterised in that** the alkali cellulose crumbs are ripened at a temperature of approx. 72°C.

11. Process in accordance with Claim 1, **characterised in that** the alkali cellulose crumbs in Step d) are ripened to a maturity of between 8° and 12° Hottenroth.

12. Process in accordance with Claim 11, **characterised in that** the alkali cellulose crumbs are ripened to a maturity of about 10° Hottenroth.

13. Process in accordance with Claim 1, **characterised in that** the wet sulphide process in Step e) is carried out in a solution containing carbon disulphide, sodium hydroxide and Berol.

14. Process in accordance with Claim 13, **characterised in that** the carbon disulphide content of the solution is between 150 and 250 g/l and the sodium hydroxide content is between 250 and 350 g/l.

15. Process in accordance with Claim 14, **characterised in that** the carbon disulphide content of the solution is between 180 and 210 g/l and the sodium hydroxide content is between 280 and 320 g/l.

16. Process in accordance with Claim 1, **characterised in that** subsequent ripening of the cellulose in Step g) is carried out to a maturity of between 8° and 12° Hottenroth.

17. Process in accordance with Claim 1, **characterised in that** the spinning solution downstream of the subsequent ripening of the cellulose and upstream of the filtration of the spinning solution is mixed with at least one other spinning solution produced using a process which comprises Steps a) to g) as described in Claim 1.

18. Process in accordance with Claim 1, **characterised in that** the temperature of the regenerating bath in Step i) is between 35°C and 45°C.

19. Process in accordance with Claim 18, **characterised in that** the temperature of the regenerating bath is approximately 40°C.

20. Process in accordance with Claim 1, **characterised in that** the regenerating bath in Step i) contains between 70 and 160 g/l, preferably between 90 and 140 g/l, and ideally approximately 120 g/l of sulphuric acid.

21. Process in accordance with Claim 1, **characterised in that** the regenerating bath in Step i) contains between 0.3 and 4 g/l, preferably between 0.5 and 2 g/l, and ideally approximately 1 g/l of zinc sulphate.

22. Process in accordance with Claim 1, **characterised in that** the regenerating bath in Step i) contains between 0.05 and 1 g/l, preferably between 0.1 and 0.7 g/l, and ideally approximately 0.4 g/l of Berol.

23. Process in accordance with Claim 1, **characterised in that** the spinnerets in Step i) are heated to keep them at a temperature of between 55°C and 75°C.

24. Process in accordance with Claim 23, **characterised in that** the spinnerets are kept at a temperature of between

24

65°C and 70°C, and preferably at approx. 67°C.

25. Process in accordance with Claim 1, **characterised in that** the spinnerets in Step i) are oval to long-slit-shaped.

26. Process in accordance with Claim 1, **characterised in that** dehydrating of the fibres in Step k) is carried out with a sulphuric acid solution which contains up to 15 g/l of sulphuric acid.

27. Process in accordance with Claim 26, **characterised in that** the sulphuric acid solution used to dehydrate the fibres contains up to 10 g/l of sulphuric acid.

28. Process in accordance with Claim 1, **characterised in that** desulphurisation of the fibres in Step l) is carried out with a sodium sulphate solution which contains between 2 and 5 g/l of sodium sulphate.

29. Process in accordance with Claim 28, **characterised in that** the sodium sulphate solution used to desulphurise the fibres contains approximately 3 g/l of sodium sulphate.

30. Process in accordance with Claim 1, **characterised in that** the twisted fibres are treated with titanium dioxide after being washed with water and before being dehydrated.

31. Process in accordance with Claim 1, **characterised in that** the predehydrating of the fibres in Step n) is carried. out with compressed air.

32. Process in accordance with Claim 1, **characterised in that** the drying of the fibres in Step o) is carried out under application of tunnel dryers.

33. Cellulose fibre, obtainable by a process in accordance with one of the Claims 1 to 32, **characterised in that** it has a microstructure with fibre-parallel lamellae, essentially contains no lignin and is substantially free of sulphuric acid and carbon disulphide.

34. Cellulose fibre in accordance with Claim 33, **characterised in that** the spacing between the fibre-parallel lamellae ranges between 1 nm and 5 μm.

35. Cellulose fibre in accordance with Claim 34, **characterised in that** the spacing between the fibre-parallel lamellae ranges between 200 nm and 1 μm.

36. Fabric comprising a backing fabric and a pile woven into the backing fabric which contains the fibres in accordance with one of the Claims 33 to 35.

37. Fabric in accordance with Claim 36, **characterised in that** the backing fabric has a lattice-like structure.

38. Fabric in accordance with Claim 36, **characterised in that** the pile forms a fibre bed of approx. 0.5 cm in height above the backing fabric.

39. Fabric in accordance with Claim 36, **characterised in that** the backing fabric contains viscose staple fibres.

40. Fabric in accordance with Claim 39, **characterised in that** the backing fabric consists exclusively of viscose staple fibres.

41. Fabric in accordance with Claim 36, **characterised in that** the pile contains oval and tape fibres.

42. Fabric in accordance with Claim 41, **characterised in that** the pile consists of 50% oval fibres and 50% tape fibres.

43. Fabric in accordance with Claim 42. **characterised in that** the pile consists of 50% of oval fibres with a count of 330 dtex F60 and 50% of tape fibres with a count of 330 dtex F80.

44. Use of the fabric in accordance with one of the Claims 36 to 43 for cleaning and decontamination applications.

45. Use of the fabric in accordance with one of the Claims 36 to 43 to reduce the surface tension of water.

**46.** Use of the fabric in accordance with one of the Claims 36 to 43 to make textiles.

**47.** Use of the fabric in accordance with one of the Claims 36 to 43 to make clothing textiles.

**48.** Use of the fabric in accordance with one of the Claims 36 to 43 to make personal hygiene articles.

**49.** Use of the fabric in accordance with one of the Claims 36 to 43 as a particle filter.

**50.** Use of the fabric in accordance with one of the Claims 36 to 43 as a condensation catalyst.

**51.** Use of the fabric in accordance with one of the Claims 36 to 43 as a floor covering.

**52.** Use of the fabric in accordance with one of the Claims 36 to 43 as a covering material.

**Revendications**

**1.** Procédé pour la fabrication d'une fibre cellulosique à partir de cellulose hydratée, qui contient les étapes suivantes :

a) la manipulation de cellulose provenant de scions de bois feuillu ou résineux d'au plus 1 an avec une solution d'hydroxide de métaux alacalins, pour obtenir une cellulose alcaline,

b) l'extraction par pression de la solution de métaux alcalins en surplus de la cellulose alcaline obtenue,

c) la fibrillation de la cellulose alcaline,

d) la pré-maturation de la cellulose alcaline fibrillée jusqu'à un degré de maturité de 5° à 30°

e) la manipulation de la cellulose selon le procédé par sulfide humide, pour convertir la cellulose en ester xanthogénique,

f) le lavage et la dilution de la cellulose convertie en ester xanthogénique avec de l'eau, pour fabriquer une solution de filage,

g) la maturation de la cellulose lavée et diluée jusqu'à un degré de maturité de 5° à 30°

h) le filtrage et ensuite la désaération de la solution de filage,

i) le pressage de la solution de filage dans un bain de régénération en utilisant des filières,

j) le filage des fibres coagulées en tordant régulierement les fibres, pour fabriquer une fibre torsadée,

k) l'égouttage des fibres tordues,

l) la désulfuration des fibres tordues,

m) le lavage à l'eau des fibres torsdues,

n) l'égouttage préalable des fibres tordues et

o) le séchage des fibres tordues.

**2.** Procédé selon l'exigence 1, **caractérisé par** ceci que la cellulose est choisie dans des scions de bois de Robinie, Teck, bois de Bongassi et bambou d'au plus 1 an.

**3.** Procédé selon l'exigence 1, **caractérisé par** ceci que la teneur en lignine du scion d'au plus 1 an ne dépasse pas les 7%, de préférence les 5% et particulièrement de préférence les 2%.

**4.** Procédé selon l'exigence 1, **caractérisé par** ceci que la solution d'hydroxyde de métaux alcalins nécessaire au traitement de la cellulose à l'étape a) utilisée est une soude caustique avec une teneur en hydroxide de sodium se trouvant entre 150 et 350 g/l.

**5.** Procédé selon l'exigence 4, **caractérisé par** ceci que la soude caustique contient environs 300 g/l d'hydroxide de sodium.

**6.** Procédé selon l'exigence 1, **caractérisé par** ceci que le traitement de la cellulose à l'étape a) est conduit à une température entre 15 et 25°C.

**7.** Procédé selon l'exigence 1, **caractérisé par** ceci que la fibrillation de la cellulose alcaline à l'étape c) comprend un broyage grossier et un broyage afiné.

**8.** Procédé selon l'exigence 1, **caractérisé par** ceci que la cellulose alcaline fibrillée est à l'étape d) maturée par une température entre 60 et 80°C.

**9.** Procédé selon l'exigence 8, **caractérisé par** ceci que la cellulose alcaline fibrillée est maturée par une température entre 65 et 75°C.

**10.** Procédé selon l'exigence 9, **caractérisé par** ceci que la cellulose alcaline fibrillée est maturée par une température d'environs 72°C.

**11.** Procédé selon l'exigence 1, **caractérisé par** ceci que la cellulose alcaline fibrillée est à l'étape d) maturée jusqu'à un degré de maturité compris entre 8 et 12.

**12.** Procédé selon l'exigence 11, **caractérisé par** ceci que la cellulose alcaline fibrillée est maturée jusqu'à un degré de maturité d'environs 10.

**13.** Procédé selon l'exigence 1, **caractérisé par** ceci que le procédé au sulfide humide à l'étape e) est effectué dans une solution de sulfure de carbone, d'hydroxide de sodium et de berol.

**14.** Procédé selon l'exigence 13, **caractérisé par** ceci que la teneur en sulfure de carbone soit comprise entre 150 et 250 g/l et celle en hydroxide de sodium entre 250 et 350 g/l.

**15.** Procédé selon l'exigence 14, **caractérisé par** ceci que la teneur en sulfure de carbone soit comprise entre 180 et 210 g/l et celle en hydroxide de sodium entre 280 et 320 g/l.

**16.** Procédé selon l'exigence 1, **caractérisé par** ceci que la rematuration de la cellulose à l'étape g) est conduite jusqu'à un degré de maturité compris entre 8 et 12.

**17.** Procédé selon l'exigence 1, **caractérisé par** ceci que la solution de filage, après la rematuration de la cellulose et avant son filtrage, est mélangée à au moins une autre solution de filage, qui aura été produite par un procédé comprenant, conformément à l'exigence 1, les étapes a) à g).

**18.** Procédé selon l'exigence 1, **caractérisé par** ceci que la température du bain de régénération à l'étape i) est comprise entre 35 et 45°C.

**19.** Procédé selon l'exigence 18, **caractérisé par** ceci que la température du bain de régénération est d'environs 40°C.

**20.** Procédé selon l'exigence 1, **caractérisé par** ceci que le bain de regénération à l'étape i) contient entre 70 et 160 g/l d'acide sulfurique, de préférence entre 90 et 140 g/l et particulièrement de préférence environs 120g/l.

**21.** Procédé selon l'exigence 1, **caractérisé par** ceci que le bain de regénération à l'étape i) contienne entre 0,3 et 4 g/l de sulfate de zinc, de préférence entre 0,5 et 2 g/l et particulièrement de préférence environs 1g/l.

**22.** Procédé selon l'exigence 1, **caractérisé par** ceci que le bain de regénération à l'étape i) contient entre 0,05 et 1 g/l de bérol, de préférence entre 0,1 et 0,7 g/l et particulièrement de préférence environs 0,4 g/l.

**23.** Procédé selon l'exigence 1, **caractérisé par** ceci que les filières à l'étape i) sont maintenues à une température comprise entre 55 et 75°C en chauffant.

**24.** Procédé selon l'exigence 23, **caractérisé par** ceci que les filières sont maintenues à une température comprise entre 65 et 70°C, de préférence à une température d'environs 67°C.

**25.** Procédé selon l'exigence 1, **caractérisé par** ceci que les filières à l'étape i) ont une forme de fente ovale jusqu'à allongée.

**26.** Procédé selon l'exigence 1, **caractérisé par** ceci que l'égouttage des fibres à l'étape k) est conduit à l'aide d'une solution d'acide sulfurique, qui contient jusqu'à 15 g/l d'acide sulfurique.

**27.** Procédé selon l'exigence 26, **caractérisé par** ceci que la solution d'acide sulfurique utilisée pour l'égouttage des fibres contient jusqu'à 10 g/l d'acide sulfurique.

**28.** Procédé selon l'exigence 1, **caractérisé par** ceci que la désulfuration des fibres à l'étape 1) est conduit à l'aide d'une solution de sulfate de sodium, qui contient de 2 g/l à 5 g/l de sulfate de sodium.

**29.** Procédé selon l'exigence 28, **caractérisé par** ceci que la solution de sulfate de sodium utilisée pour la désulfuration des fibres contient jusqu'à 3 g/l sulfate de sodium.

**30.** Procédé selon l'exigence 1, **caractérisé par** ceci que la fibre torsadée est préparée après le lavage avec de l'eau et avant l'égouttage avec du dioxyde de titan.

**31.** Procédé selon l'exigence 1, **caractérisé par** ceci que le préessorage de la fibre à l'étape n) est conduit avec de l'air sous pression.

**32.** Procédé selon l'exigence 1, **caractérisé par** ceci que le séchage de la fibre à l'étape o) est conduit en utilisant un sécheur à tunnel.

**33.** Une fibre celullosique, disponible selon un procédé suivant une des exigences 1 à 32, **caractérisée par** ceci qu'elle a une microstructure avec des lamelles parallèles à la fibre, qu'elle ne contient pour l'essentiel pas de lignine et qu'elle est libre de tout acide sulfurique et sulfure de carbone.

**34.** Une fibre cellulosique selon l'exigence 33, **caractérisée par** ceci que les lamelles parallèles à la fibre sont à une distance de 1nm à 5 μm l'une de l'autre.

**35.** Une fibre cellulosique selon l'exigence 34, **caractérisée par** ceci que les lamelles parallèles à la fibre sont à une distance de 200 nm à 1 μm l'une de l'autre.

**36.** Le tissu est constitué d'une sous couche et d'une flore lui étant attachée, qui contient la fibre répondant à l'une des exigences 33 à 35.

**37.** Tissu selon l'exigence 36, **caractérisé par** ceci que la sous couche possède une structure en grillage.

**38.** Tissu selon l'exigence 36, **caractérisé par** ceci que la flore forme une pelouse de fibres d'environs 0,5 cm de long sur la sous couche.

**39.** Tissu selon l'exigence 36, **caractérisé par** ceci que la sous couche contient des fibres textiles en viscose.

**40.** Tissu selon l'exigence 39, **caractérisé par** ceci que la sous couche se compose de fibres textiles en viscose.

**41.** Tissu selon l'exigence 36, **caractérisé par** ceci que la flore comprend des fibres ovales et allongées.

**42.** Tissu selon l'exigence 41, **caractérisé par** ceci que la flore se compose de 50% de fibres ovales et 50% de fibres allongées.

**43.** Tissu selon l'exigence 42, **caractérisé par** ceci que la flore se compose de 50% de fibres ovales de dimension 330 dtex F 60 et 50% de fibres allongées de dimension 330 dtex F 80.

44. Utilisation du tissu selon une des exigences 36 à 43 pour le nettoyage et la décontamination.

45. Utilisation du tissu selon une des exigences 36 à 43 pour réduire la tension superficielle de l'eau.

46. Utilisation du tissu selon une des exigences 36 à 43 pour la fabrication de textiles.

47. Utilisation du tissu selon une des exigences 36 à 43 pour la fabrication de matériaux de revêtement.

48. Utilisation du tissu selon une des exigences 36 à 43 pour la production de produits d'hygiène.

49. Utilisation du tissu selon une des exigences 36 à 43 comme filtre à particules.

50. Utilisation du tissu selon une des exigences 36 à 43 comme catalyseur de condensation.

51. Utilisation du tissu selon une des exigences 36 à 43 comme revêtement du sol.

52. Utilisation du tissu selon une des exigences 36 à 43 comme matériau de recouverment.

Figuren 1 bis 6

REM-Aufnahmen der Proben

## Figuren 7 bis 12

REM-Aufnahmen von Dünnschnitten (ca. 3 μm dick)

Figuren 13 bis 15

## Figur 16

### Velours-Gewebe (M-2/250)

Gewebebild
(Aufsicht)

Gewebebild
(Schnitt)

Florfaden F 60
rundförmig

Florfaden F 80
bändchenförmig

Viskose-Florfaden
330 dtex f 60
rundförmig

Viskose-Granolfaden
(Spinnfaser) Nm 50/2

Viskose-Granolfaden
(Spinnfaser) Nm 50/2

Viskose-Florfaden
330 dtex F 80
bändchenförmig

Viskose-Schußfaden
(Spinnfaser) Nm 28/1

Grundgewebe

Floroberfläche

EP 1 012 359 B1

Figuren 17 und 18

34

Figuren 19 und 20

```
x20000        2μm                1.18kV        3mm
#960035    STAPHYLOKOKKEN        LEO 982*GEMINI
```

```
x30000        1μm                1.18kV        4mm
#960035    STAPHYLOKOKKEN        LEO 982*GEMINI
```

Figur 21

Gewebetücher

| x | y | y | y | x |

Überlauf

Ablauf

x,y: Abstände der Gewebetücher

## Figur 22

DIGITALANZEIGE DER OBERFLÄCHENSPANNUNG IN mN/m

70,4 mN/m

PLATINRING

WASSERPROBE

PROBENGEFÄSS (GLAS)

VERTIKAL BEWEGLICHER TISCH

NULLANZEIGE

WIEDERHOLUNGS-TASTE

KORREKTURKNÖPFE

TENSIOMETER ZUR MESSUNG DER OBERFLÄCHENSPANNUNG

## Figuren 23 und 24

OBERFLÄCHENENTSPANNENDE WIRKUNG DES GEWEBES L01
IN ABHÄNGIGKEIT DER SPÜLUNGEN DES GEWEBES

OBERFLÄCHENENTSPANNENDE WIRKUNG DES GEWEBES L02
IN ABHÄNGIGKEIT DER SPÜLUNGEN DES GEWEBES

## Figuren 25 und 26

OBERFLÄCHENENTSPANNENDE WIRKUNG DES GEWEBES S10
IN ABHÄNGIGKEIT DER SPÜLUNGEN DES GEWEBES

OBERFLÄCHENENTSPANNENDE WIRKUNG DES
DOPPELSEITIGEN GEWEBES L01

## Figuren 27 und 28

OBERFLÄCHENENTSPANNENDE WIRKUNG DES
DOPPELSEITIGEN GEWEBES L02

OBERFLÄCHENENTSPANNENDE WIRKUNG DES
DOPPELSEITIGEN GEWEBES S10

Figuren 29 und 30

OBERFLÄCHENSPANNUNG MIT IM
WASSER VERBLEIBENDEN GEWEBEN

OBERFLÄCHENENTSPANNENDE WIRKUNG DES GEWEBES L01
VOR BZW. NACH DER TROCKNUNGSPHASE (MITTELWERTE)

41

## Figuren 31 und 32

Gewebe

Schiefe Ebene

45 °

Versuchsaufbau zur Messung des Wasseraufnahmevermögens

1 µm

2 µm

4 µm

80 µm